# EUROPEAN PATENT APPLICATION

(11) **EP 2 385 128 A1**
(43) Date of publication of application: **09.11.2011**
(21) Application number: 10004896.6
(22) Date of filing: 07.05.2010
(51) Int. Cl.: C12N 15/82, C12N 15/29, A01H 5/00

(54) **Modulation of germination, elongation growth and flowering time in plants**

(71) Applicant: Technische Universität München, 80333 München (DE)
(72) Inventor: The designation of the inventor has not yet been filed
(74) Representative: Engelhard, Markus

(57) **Abstract**

The present invention relates to a method for modulating plant developmental processes. In particular, the present invention relates to a method for inhibiting or stimulating germination, elongation growth and flowering time in plants by modulating the activity of genes/proteins that function downstream of the gibberellic acid (GA) signal transduction pathway. The present invention also relates to plants obtained by this method, to nucleic acids used in this method, as well as to plant cells and plants comprising the same.

## Description

The present invention relates to a method for modulating plant developmental processes. In particular, the present invention relates to a method for inhibiting or stimulating germination, elongation growth and flowering time in plants by modulating the activity of genes/proteins that function downstream of the gibberellic acid (GA) signal transduction pathway. The present invention also relates to plants obtained by this method, to nucleic acids used in this method, as well as to plant cells and plants comprising the same.

Plant growth can be roughly divided into four phases. Prior and during germination the seed integrates external factors (temperature, availability of water, light, day length etc.) to decide whether or not to germinate. After germination and during vegetative growth, the plant accumulates biomass and nutrients by photosynthesis and nutrient uptake from the soil. Through the integration of a range of physiological parameters as well as environmental inputs (nutrients, temperature, availability of water, light, day length etc.) the plant then decides whether or not to enter reproductive development, which is characterized by flower formation, pollination, fertilization and fruit formation. The moment of transition from the vegetative phase to the reproductive phase is termed flowering time. Flowering time can be measured by counting the number of days until bolting (inflorescence stem elongation) or flower formation, or alternatively by counting the number of vegetative leaves produced until bolting or flower formation. In many plants, vegetative growth stops after the onset of reproductive growth, and the nutrients accumulated during the vegetative phase are used for fruit formation.

In particular with respect to agricultural applications, germination, elongation growth and flowering time are important parameters of plant growth. For example, seeds of crop and horticultural plants should not germinate after seed maturation or during seed/fruit storage, on the other hand they should germinate rapidly and uniformly after sowing in order to allow for optimal land use. Equally important is a controlled and coordinated flowering time of agronomically important plants. Temporal coordination of flowering time is also important for agricultural crops, since the extent of fruit formation and, ultimately, crop yield depend on the amount of nutrients that have accumulated during the vegetative phase. Furthermore, optimal crop yield relies on appropriate plant architecture and stability, e.g. the formation of a robust stem and lodging resistance during vegetative growth, which allows to bear the heavy fruit of high-yielding varieties.

In the past, selective breeding of agricultural crops and horticultural plants has allowed to adopt germination behaviour, vegetative growth, flowering time as well as fruit formation to the needs of farmers, producers and consumers, particularly with regard to the specific environmental conditions of the respective locations. For example, an important advance was the use of *Rht* dwarfing alleles in wheat breeding in international breeding programs after the second world war, which has led to increases in crop yields that were later termed the "green revolution". The systematic use of these dwarfing alleles appears to be largely responsible for the dramatic world-wide increases in wheat production - and the concomitant decline in wheat prices - since the 1950s. The dwarfed wheat plants were more resistant against stem lodging and were able to bear the ears of high-yielding varieties. The use of dwarfing genes facilitated similar advances in the breeding of rice. As in wheat, the dwarfing of rice and a similar effect on rice yield were achieved by introducing mutants of the gibberellic acid (GA) pathway.

GA is one of several known plant growth hormones. In wild-type plants, GA promotes a number of essential developmental processes, among them germination, elongation growth and flowering time (Schwechheimer, Curr Opin Plant Biol 2008; Schwechheimer and Willige, Curr Opin Plant Biol 2009). GA-insensitive plants exhibit delayed germination, dwarfism and delayed flowering. The molecular identity of the wheat *Rht* dwarfing alleles has been revealed, and the mode of action has been found to be related to the GA signalling pathway (Peng et al., Nature 1999). The wheat genes/proteins causing dwarfing are mutant forms of so-called DELLA proteins with a deletion or mutation of the N-terminal DELLA domain. In the wild-type DELLA protein, the DELLA domain is required for DELLA protein degradation in response to GA. Proteins with a mutation in the DELLA domain such as those encoded by the wheat *Rht* dwarfing alleles cannot be degraded in response to GA, resulting in an at least partial GA-insensitivity of the respective plants (Willige et al., Plant Cell 2007). This physiological and biochemical mode of action appears to apply to all DELLA proteins and their DELLA domain-mutant forms (Willige et al., Plant Cell 2007).

DELLA proteins are conserved in all plant organisms and - in the absence of GA - repress the binding of transcription regulators of the conserved PHYTOCHROME INTERACTING FACTORS (PIFs) protein family to their cognate promoter elements. Since DELLA proteins are degraded in response to GA, PIFs can subsequently regulate the expression of their target genes. Thus, DELLA proteins regulate the expression of GA target genes indirectly, whereas PIF proteins regulate their expression directly. These target genes, whose identities and functions are largely unknown, are expected to be responsible for specific aspects of the GA response.

Therefore, it was an object of the present invention to identify DELLA/PIF-regulated target genes (genes being dependent on DELLA-mediated protein degradation and PIF activity) and to use these genes and their homologues, the corresponding gene products, i.e. polypeptides, and fragments thereof for modulating GA-mediated plant developmental processes, in particular germination, elongation growth and flowering time in plants.

The objects of the present invention are solved by the genes GNC (*GATA, NITRATE-INDUCIBLE, CARBON-METABOLISM-INVOLVED;* AT5G56860) and GNL/CGAI (*GNC-LIKEICYTOKININ-RESPONSIVE GATA FACTOR 1;* AT4G26150; herein referred to as GNL), from *Arabidopsis thaliana,* as well as their homologues. These genes code for transcription factors that act downstream of the known DELLA/PIF regulators.

The objects of the present invention are solved by a method for modulating (i.e. inhibiting or stimulating) at least one plant developmental process selected from germination, elongation growth and flowering time, said method comprising:
modulating (i.e. inhibiting or stimulating) the activity of at least one polypeptide in a plant or in a plant cell, said polypeptide comprising
   a GATA DNA-binding domain and
   a conserved domain comprising the amino acid sequence
   A - A - Xₙₒₙ₋ₚₒₗₐᵣ Or X_{uncharged} polar - L - L - M - X - L - S ,
   wherein Xₙₒₙ-ₚₒₗₐᵣ is any amino acid having a non-polar side chain, X_{uncharged} polar is any amino acid having an uncharged polar side chain and X is any amino acid.

Thus, the method according to the present invention results in plants characterized by the modulation (i.e. inhibition or stimulation) of at least one developmental process selected from germination, elongation growth and flowering time as compared to wild-type plants. The term "inhibition", as used herein, is meant to refer to a complete inhibition (i.e. inactivation/stop) or to a partial inhibition (resulting in a decrease/reduction/delay). The phrase "modulating the activity of at least one polypeptide in a plant cell" is meant to refer to a scenario, where the activity of said at least one polypeptide is first modulated in one or several plant cells, which are then subsequently regenerated into whole plants. Therefore, the method according to the present invention can also be regarded as a method for producing a plant being characterized by the modulation (i.e. inhibition or stimulation) of at least one developmental process selected from germination, elongation growth and flowering time. In a preferred embodiment, all three developmental processes are modulated at the same time.

GATA DNA-binding domains are characterized by their binding to the DNA sequence (T/A)GATA(A/G) as well as by the presence of a zinc finger motif having the amino acid sequence C ― X₂ ― C ― X₁₇₋₂₀ ― C ― X₂ ― C, wherein X refers to any amino acid and the subscript numbers refer to the number of amino acids. In a preferred embodiment, said zinc finger motif is followed by basic region, comprising at least 4 amino acid residues selected from R and K.

In one embodiment, said conserved domain comprises the amino acid sequence
A ― A ― Xₙₒₙ₋ₚₒₗₐᵣ ― L ― L ― M ― X ― L ― S,
herein Xₙₒₙ₋ₚₒₗₐᵣ is any amino acid having a non-polar side chain and X is any amino acid.

In one embodiment, said conserved domain comprises the amino acid sequence
Xₚₒₗₐᵣ ― Xₚₒₗₐᵣ ― A ― A ― Xₙₒₙ₋ₚₒₗₐᵣ Or X_{uncharged polar} ― L ― L ― M ― X ― L ― S,
wherein Xₚₒₗₐᵣ is any amino acid having a polar side-chain, Xₙₒₙ₋ₚₒₗₐᵣ is any amino acid having a non-polar side chain, X_{uncharged} polar is any amino acid having an uncharged polar side chain and X is any amino acid.

In one embodiment, said conserved domain comprises the amino acid sequence
Xₚₒₗₐᵣ ― Xₚₒₗₐᵣ ― A ― A ― Xₙₒₙ₋ₚₒₗₐᵣ ― L ― L ― M ― X ― L ― S,
wherein Xₚₒₗₐᵣ is any amino acid having a polar side-chain, Xₙₒₙ₋ₚₒₗₐᵣ is any amino acid having a non-polar side chain and X is any amino acid.

The term "polar side-chain", as used herein, refers to both charged and uncharged polar side-chains. Amino acids having a polar (i.e. hydrophilic) side-chain include S, T, N, Q, Y, C, K, R, H, D and E, with E, D, T, K, R and Q being particularly preferred according to the present invention. Amino acids having an uncharged polar side chain include S, T, N, Q, Y, C, with S, T and Y being preferred, and T being particularly preferred according to the present invention.

Amino acids having a non-polar (i.e. hydrophobic) side chain include G, A, V, L, I, M, P, F, W, with I, M,V and F being particularly preferred according to the present invention.

In one embodiment, said conserved domain comprising one of the above amino acid sequences is located at the C-terminus of said polypeptide, i.e. it is a C-terminal domain. Preferably, said C-terminal domain is located within the last 60, more preferably within the last 50, even more preferably within the last 40 amino acids of said polypeptide.

In one embodiment, said polypeptide
(i) has an amino acid sequence which is SEQ ID NO: 1 or SEQ ID NO: 2 or a fragment thereof;
(ii) is a homologue to SEQ ID NO: 1 or SEQ ID NO: 2, wherein, preferably, said homologue has an amino acid sequence being selected from SEQ ID NO: 3-29; or
(iii) has an amino acid sequence which is at least 50%, preferably at least 60%, more preferably at least 70%, even more preferably at least 80%, even more preferably at least 90%, and most preferably at least 95% identical to any of SEQ ID NO: 1-29 or a fragment thereof.

The reference to a sequence *a* which is "at least x% identical" to a sequence *b* is meant to refer to a scenario, wherein at least x% of the amino acid residues of sequence *b* are also present in sequence *a* in corresponding positions.

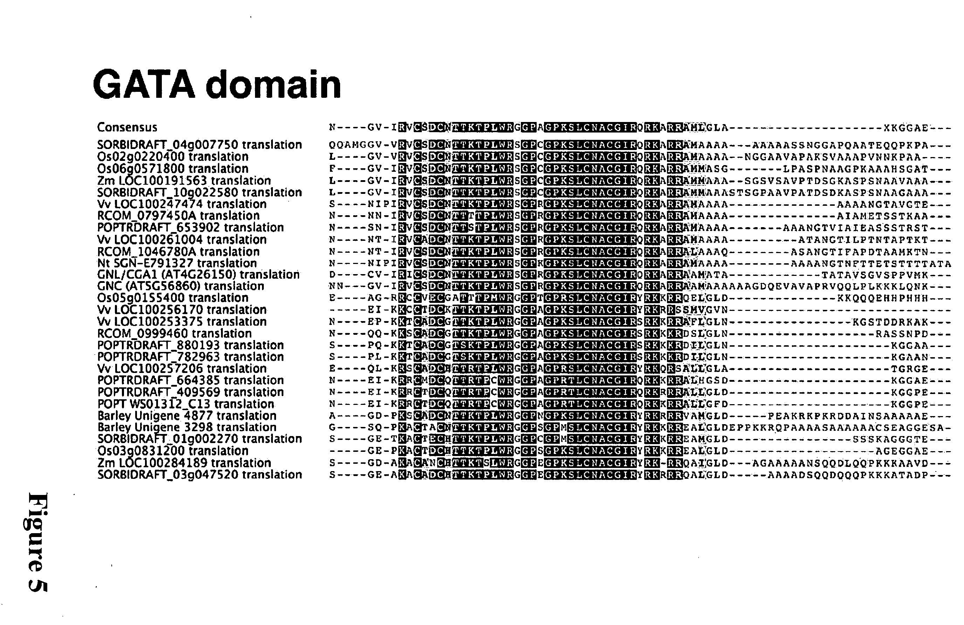

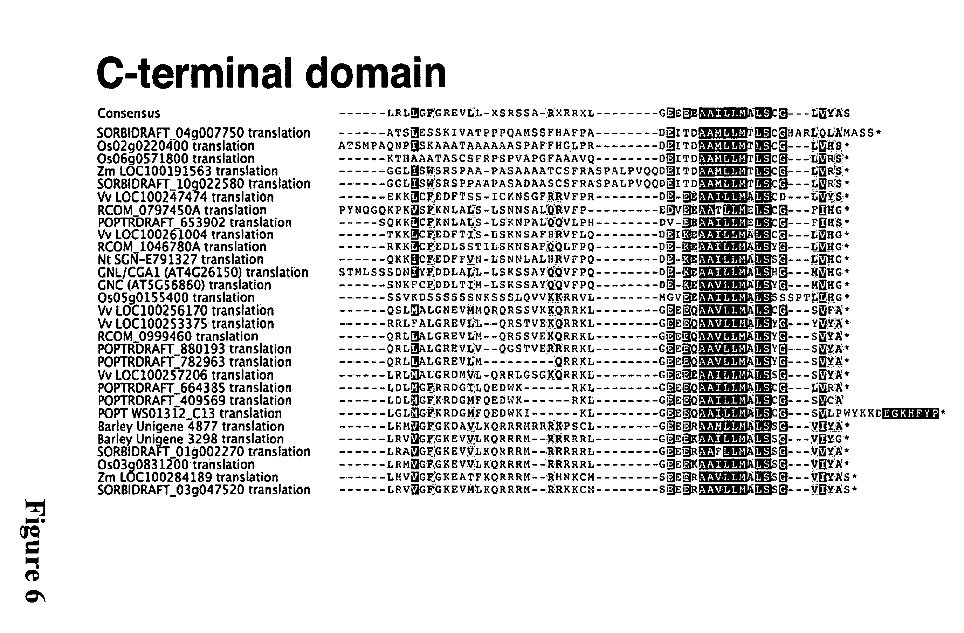

The term "homologue" to a polypeptide *a* is meant to refer to a polypeptide *b* that has the same physiological function as polypeptide *a*. Homologues may exist both in *Arabidopsis thaliana* and in plant species other than *Arabidopsis thaliana.* Homologues are typically characterized by the presence of defined sequence conservation and can be identified by BLAST searches, sequence alignments and other bioinformatics tools and algorithms known to the skilled person. The homologues according to the present invention are characterized by the presence of a GATA DNA-binding domain and a conserved, preferably C-terminal domain as defined above (see also **Figure 5** and **Figure 6**).

The activity of the polypeptides as defined above (herein also referred to as "polypeptides according to the present invention") can be modulated both on the gene level (DNA/RNA) and the protein level.

In one embodiment, said modulating the activity of said polypeptide results in a decreased activity of said polypeptide. The decrease in activity of the polypeptides according to the present invention (or their inactivation) results in an accelerated germination, increased elongation growth and early flowering time. In other words, inhibition of the activity of the polypeptides according to the present invention results in the stimulation of germination, flowering time and elongation growth.

In one embodiment, the activity of said polypeptide is decreased by at least 10%, more preferably by at least 20%, more preferably by at least 30%, more preferably by at least 40%, more preferably by at least 50%, more preferably by at least 60%, more preferably by at least 70%, more preferably by at least 80%, even more preferably by at least 90%, as compared to the normal activity of said polypeptide in the respective wild-type plant or plant cell. Most preferably, the activity of said polypeptide is completely abolished (also referred to as inactivation).

In one embodiment, said modulating the activity of said polypeptide resulting in a decreased activity of said polypeptide occurs by one of the following:
(i) producing a mutant nucleic acid coding for a version of said polypeptide having decreased activity, preferably a non-functional version of said polypeptide, through insertion, deletion or mutation of at least one nucleotide in the nucleic acid coding for said polypeptide, and introducing said mutant nucleic acid into a plant cell;
(ii) producing a mutant nucleic acid coding for a version of said polypeptide having reduced expression, through insertion, deletion, mutation of at least one nucleotide at a position 1-500 nucleotides, preferably 1-100 nucleotides upstream of the transcription start site of the nucleic acid coding for said polypeptide, and introducing said mutant nucleic acid into a plant cell;
(iii) producing a mutant nucleic acid by placing the nucleic acid coding for said polypeptide under the control of a regulatory sequence that switches off the expression of said nucleic acid coding for said polypeptide, and introducing said mutant nucleic acid into a plant cell;
(iv) producing a nucleic acid coding for a polypeptide that represses the transcription of the nucleic acid coding for said polypeptide, preferably by binding to the upstream regulatory region of the nucleic acid coding for said polypeptide, and introducing said nucleic acid into a plant cell;
(v) producing, within a plant cell, a mutant nucleic acid coding for a version of said polypeptide having decreased activity, preferably a non-functional version of said polypeptide, or a version of said polypeptide having reduced expression, by exposing said plant cell to a mutagenic substance, such as ethyl methane sulfonate, or by exposing said plant cell to mutagenic radiation, such as UV-radiation or X-rays, and identifying the resultant mutant nucleic acid by gene sequencing, by the recognition of mismatch hybridizations by TILLING, or by related techniques;
(vi) introducing small regulatory RNAs directed at the nucleic acid coding for said polypeptide or at its transcript into a plant cell;
(vii) introducing antisense nucleic acid molecules complementary to the nucleic acid coding for said polypeptide into a plant cell;
(viii) introducing sense nucleic acid molecules homologous to the nucleic acid coding for said polypeptide into a plant cell (co-suppression); or
(ix) expressing a dominant-negative fragment of said polypeptide in a plant cell.

Targeting Induced Local Lesions IN Genomes (TILLING) allows directed identification of mutations in a specific gene (Slade and Knauf, Transgenic Res 2005). The method combines a standard technique of mutagenesis employing a chemical mutagen such as ethyl methane sulfonate (EMS) or mutagenic radiation with a sensitive DNA screening-technique that allows to identify (single) base mutations (point mutations) in a target gene within a large non-altered gene population. TILLING is reliable, applicable to any plant and it does not require transgenic or cell culture manipulations. Plants generated by mutagenesis, e.g. EMS mutagenesis, are by definition not genetically modified organisms.

Small regulatory RNAs are generally used to downregulate transcription or protein translation of a specific gene. Specific small regulatory RNAs suitable for this purpose can be designed by someone skilled in the art without undue experimentation. Techniques for introducing small regulatory RNAs into a plant cell are also known to a skilled person.

In one embodiment, said small regulatory RNAs are selected from the group comprising miRNAs and siRNAs. MiRNAs and siRNAs are small regulatory RNAs that pair to their target mRNA and then prevent it from being used as a translation template, e.g. by inducing cleavage of the mRNA.

In a preferred embodiment, said small regulatory RNAs are miRNAs.

In another embodiment of the present invention, said modulating the activity of said polypeptide results in an increased activity of said polypeptide. The increase in activity of the polypeptides according to the present invention results in delayed germination, in delayed flowering time as well as in reduced elongation growth. In other words, stimulation of the activity of the polypeptides according to the present invention results in inhibition of germination, flowering time and elongation growth.

In one embodiment, the activity of said polypeptide is increased by at least 10%, more preferably by at least 20%, more preferably by at least 30%, more preferably by at least 40%, more preferably by at least 50%, more preferably by at least 60%, more preferably by at least 70%, more preferably by at least 80%, more preferably by at least 90%, even more preferably by at least 100%, as compared to the normal activity of said polypeptide in the respective wild-type plant or plant cell.

In one embodiment, said modulating the activity of said polypeptide resulting in an increased activity of said polypeptide occurs by one of the following:
(i) producing a mutant nucleic acid coding for a version of said polypeptide having increased activity or a version of said polypeptide having increased expression, through insertion, deletion, mutation of at least one nucleotide in the nucleic acid coding for said polypeptide or at a position 1-500 nucleotides, preferably 1-100 nucleotides upstream of the transcription start site of the nucleic acid coding for said polypeptide, and introducing said mutant nucleic acid into a plant cell;
(ii) producing a mutant nucleic acid, within a plant cell, by placing the nucleic acid coding for said polypeptide under the control of a regulatory sequence that increases expression of said nucleic acid coding for said polypeptide;
(iii) producing, within a plant cell, a mutant nucleic acid coding for a version of said polypeptide having increased activity or a version of said polypeptide having increased expression, by exposing said plant cell to a mutagenic substance, such as ethyl methane sulfonate, or by exposing said plant cell to mutagenic radiation, such as UV-radiation or X-rays, and identifying the resultant mutant nucleic acid by gene sequencing, by the recognition of mismatch hybridizations by TILLING, or by related techniques;
(iv) misexpressing the nucleic acid coding for said polypeptide in a plant cell;
(v) overexpressing said polypeptide in a plant cell;
(vi) producing a nucleic acid coding for a polypeptide that increases the transcription of the nucleic acid coding for said polypeptide, preferably by binding to the upstream regulatory region of the nucleic acid coding for said polypeptide, and introducing said nucleic acid into a plant cell; or
(vii) producing a mutant nucleic acid coding for a version of said polypeptide having increased protein stability.

Method (ii) includes methods such as activation tagging, which is a method for generating dominant mutations in plants or plant cells by random insertion of a T-DNA carrying constitutive enhancer elements (Tani et al., Funct Integr Genomics 2004). The method may utilize a T-DNA sequence that contains four tandem copies of the cauliflower mosaic virus (CaMV) 35S enhancer sequence. This element enhances the expression of neighbouring genes on either side of the randomly integrated T-DNA tag, also at a distance, resulting in gain-of-function phenotypes.

The term "misexpressing" refers to a method, wherein the expression of said nucleic acid is uncoupled from the normal regulatory mechanisms of the plant cell, e.g. by placing said nucleic acid under control of another promoter, which may be achieved using transgene technologies or by inducing genome instability and subsequently DNA repair using mutagenic substances.

Methods for overexpressing genes and, thus, the corresponding polypeptides or fragments thereof in plants or plant cells are known to a person skilled in the art. Preferably, the nucleic acid coding for the gene to be overexpressed is expressed under the control of a regulatory region (promoter, enhancer) that allows for the high level expression of said nucleic acid. The polypeptides or their fragments can be overexpressed by themselves or fused to other proteins such as GFP and YFP, or epitope tags such as MYC, HA and T7.

In one embodiment, said nucleic acid coding for said polypeptide has a nucleic acid sequence which is
(i) any of SEQ ID NO: 30-58 or a fragment thereof; or
(ii) at least 50%, preferably at least 60%, more preferably at least 70%, even more preferably at least 80%, even more preferably at least 90%, and most preferably at least 95% identical to any of SEQ ID NO: 30-58 or a fragment thereof.

In one embodiment of the present invention, the activity of more than one polypeptide is modulated at the same time. For example, both the activity of GNC and of GNL (SEQ ID NOs: 1 and 30 and SEQ ID NOs: 2 and 31) can be inhibited (i.e. decreased or inactivated) in a plant or plant cell, resulting in a double mutant with an even stronger phenotype than that of the single mutants. Thus, the effects obtained by modulating the activity of the polypeptide according to the present invention can be enhanced by modulating, i.e. inhibiting or stimulating, the activity of more than one polypeptide according to the present invention at the same time, i.e. in the same plant or plant cell.

In one embodiment, the method further comprises culturing said plant or said plant cell.

The objects of the present invention are also solved by a mutant nucleic acid of a nucleic acid coding for a polypeptide as defined above, the expression of which mutant nucleic acid in a plant or in a plant cell results in less activity or more activity of said polypeptide.

In one embodiment, said mutant nucleic acid has an insertion, deletion or mutation of at least one nucleotide in the nucleic acid coding for said polypeptide, or said mutant nucleic acid has an insertion, deletion or mutation of at least one nucleotide upstream of the nucleic acid coding for said polypeptide.

In one embodiment, the expression of said mutant nucleic acid in a plant or in plant cell results in at least 10%, more preferably at least 20%, more preferably at least 30%, more preferably at least 40%, more preferably by least 50%, more preferably by least 60%, more preferably at least 70%, more preferably at least 80%, even more preferably at least 90%, most preferably in a completely abolished activity of said polypeptide in said plant or in said plant cell, as compared to the normal activity of said polypeptide in the respective wild-type plant or plant cell, or
in at least 10%, more preferably at least 20%, more preferably at least 30%, more preferably at least 40%, more preferably at least 50%, more preferably at least 60%, more preferably at least 70%, more preferably at least 80%, more preferably at least 90%, even more preferably at least 100% more activity of said polypeptide in a plant or in a plant cell, as compared to the normal activity of said polypeptide in the respective wild-type plant or plant cell.

Preferably, said insertion, deletion or mutation of at least one nucleotide upstream of the nucleic acid coding for said polypeptide is located at a position 1-500 nucleotides, preferably 1-100 nucleotides upstream of the nucleic acid coding for said polypeptide.

The objects of the present invention are also solved by an overexpression vector comprising a nucleic acid coding for a polypeptide as defined above. In one embodiment, said overexpression vector further comprises a regulatory region (promoter, enhancer) that allows for the high level expression of said nucleic acid. In one embodiment, said overexpression vector further comprises at least one nucleic acid coding for a protein such as GFP and YFP, or an epitope tags such as MYC, HA and T7.

In one embodiment, said nucleic acid coding for said polypeptide has a nucleic acid sequence which is
(i) any of SEQ ID NO: 30-58 or a fragment thereof; or
(ii) at least 50%, preferably at least 60%, more preferably at least 70%, even more preferably at least 80%, even more preferably at least 90%, and most preferably at least 95% identical to any of SEQ ID NO: 30-58 or a fragment thereof.

The mutant nucleic acids and the overexpression vector can be introduced into a plant cell through standard techniques, such as *Agrobacterium tumefaciens*-mediated transformation, biolistic transformation or other types of transformation. The transgenic plant cells thus obtained can then subsequently be regenerated into whole plants.

Accordingly, the objects of the present invention are also solved by a method for producing transgenic plants or plant cells, said method comprising
(i) producing a mutant nucleic acid or an overexpression vector as defined above;
(ii) introducing said mutant nucleic acid or said overexpression vector into plant cells; and, optionally,
(iii) regenerating said plant cells, into which said mutant nucleic acid or said overexpression vector have been introduced, into whole plants.

In one embodiment, such transgenic plants are characterized by a decreased (inhibited) or an increased (stimulated) activity of the polypeptide according to the present invention encoded by said mutant nucleic acid, thereby inhibiting or stimulating germination, elongation growth and flowering time. In particular, a decreased activity of the polypeptide according to the present invention results in an accelerated germination, increased elongation growth and early flowering time. An increased activity of the polypeptide according to the present invention results in delayed germination, in delayed flowering time as well as in reduced/decreased elongation growth. These effects can be enhanced by modulating, i.e. increasing (stimulating) or decreasing (inhibiting), the activity of more than one polypeptide according to the present invention at the same time (e.g. by producing more than one mutant nucleic acid or overexpression vector as defined above and introducing said mutant nucleic acids or said overexpression vectors into the same plant cells).

The objects of the present invention are further solved by a nucleic acid consisting of 15 to 21 or 21 to 50, or 50 to 100, or 100 to 150, or 150 to 200, preferably 15 to 21 or 21 to 50, contiguous nucleotides, preferably ribonucleotides, of the nucleic acid coding for a polypeptide as defined above.

In one embodiment, said nucleic acid coding for said polypeptide has a nucleic acid sequence which is
(i) any of SEQ ID NO: 30-58 or a fragment thereof; or
(ii) at least 50%, preferably at least 60%, more preferably at least 70%, even more preferably at least 80%, even more preferably at least 90%, and most preferably at least 95% identical to any of SEQ ID NO: 30-58 or a fragment thereof.

The objects of the present invention are also solved by a plant cell comprising a nucleic acid as defined above. The term "nucleic acid" also refers to an overexpression vector as defined above.

In one embodiment, said plant cell originates from a species being selected from *Arabidopsis thaliana, Hordeum vulgare, Oryza sativa, Populus trichocarpa, Populus trichocarpa x Populus deltoides, Ricinus communis, Sorghum bicolor, Vitis vinifera, Zea mays.*

The objects of the present invention are further solved by a plant comprising one or several plant cells as defined above.

The objects of the present invention are also solved by a plant characterized by the modulation (i.e. inhibition or stimulation) of at least one developmental process selected from germination, elongation growth and flowering time as compared to a wild-type plant, obtained by the method as defined above.

In one embodiment, said plant originates from a species being selected from *Arabidopsis thaliana, Hordeum vulgare, Oryza sativa, Populus trichocarpa, Populus trichocarpa x Populus deltoides, Ricinus communis, Sorghum bicolor, Vitis vinifera, Zea mays.*

The objects of the present invention are also solved by seeds derived from a plant as defined above.

The inventors have surprisingly found that it is possible to modulate (i.e. to inhibit or stimulate) germination, elongation growth and flowering time in the model plant *Arabidopsis thaliana* by modulating the activity of GNC and/or GNL, which function downstream of GA signalling. More specifically, genetic analyses of loss-of function mutants and overexpression lines performed by the inventors revealed that GNC and GNL are functionally redundant repressors of germination, flowering time and elongation growth. Furthermore, the inventors have identified several homologues of GNC and GNL from other plants than *Arabidopsis thaliana,* which are characterized by the strong conservation of two particular sequence motifs: a GATA DNA-binding domain and a conserved domain comprising one of the above amino acid sequences, the latter of which is preferably located at the C-terminus of the polypeptides. It is very likely that the results obtained for GNC and GNL from *Arabidopsis thaliana* can be extrapolated to their homologues.

The present invention allows to uncouple the control of the plant developmental processes of germination, flowering time and elongation growth from the control by the plant hormone GA, i.e. they can be controlled independently from the presence or absence of GA. Since the genes according to the present invention, i.e. GNC, GNL and their homologues, appear to function downstream of the GA signal transduction pathway, their modulation (i.e. their inhibition or stimulation) may be more specific and is likely to cause fewer unwanted side effects than that of upstream genes/proteins. Furthermore, GNC and GNL seem to have redundant function, allowing the creation of double mutants with an even stronger phenotype.

Reference is made to the sequences in the enclosed sequence listing, wherein
**SEQ ID NO: 1** is the amino acid sequence, **SEQ ID NO: 30** is the DNA sequence of GNC *(GATA, NITRATE-INDUCIBLE, CARBON-METABOLISM-INVOLVED;* AT5G56860; Accession Nos. NP_200497 and NM_125069) from *Arabidopsis thaliana;*
**SEQ ID NO: 2** is the amino acid sequence, **SEQ ID NO: 31** is the DNA sequence of GNL/CGA1 *(GNC-LIKEICYTOKININ-RESPONSIVE GATA FACTOR 1;* AT4G26150; Accession Nos. NP_194345 and NM_118748) from *Arabidopsis thaliana,* herein referred to as GNL;
**SEQ ID NO: 3** is the amino acid sequence, **SEQ ID NO: 32** is the DNA sequence of Barley_Unigene 3298 (Assembly #35) from *Hordeum vulgare.*
**SEQ ID NO: 4** is the amino acid sequence, **SEQ ID NO: 33** is the DNA sequence of Barley_Unigene 4877 (Assembly #35) from *Hordeum vulgare.*
**SEQ ID NO: 5** is the amino acid sequence, **SEQ ID NO: 34** is the DNA sequence of Nt SGN-E791327 from *Nicotiana tabacum.*
**SEQ ID NO: 6** is the amino acid sequence, **SEQ ID NO: 35** is the DNA sequence of Os02g0220400 (Accession Nos. NP_001052851 and NM_001046316) from *Oryza sativa.*
**SEQ ID NO: 7** is the amino acid sequence, **SEQ ID NO: 36** is the DNA sequence of Os03g0831200 (Accession Nos. NP_001051792 and NM_00105 8327) from *Oryza sativa.*
**SEQ ID NO: 8** is the amino acid sequence, **SEQ ID NO: 37** is the DNA sequence of OsO5g0155400 (Accession Nos. NP_001054691 and NM_001061226) from *Oryza sativa.*
**SEQ ID NO: 9** is the amino acid sequence, **SEQ ID NO: 38** is the DNA sequence of Os06g0571800 (Accession Nos. NP_001057917 and NM_001064452) from *Oryza sativa.*
**SEQ ID NO: 10** is the amino acid sequence, **SEQ ID NO: 39** is the DNA sequence of POPT_WS01312_C13 (Accession No. ABK96226) from *Populus trichocarpa* x *Populus deltoides.*
**SEQ ID NO: 11** is the amino acid sequence, **SEQ ID NO: 40** is the DNA sequence of POPTRDRAFT 409569 (Accession Nos. XP_002302743 and XM_002302707) from *Populus trichocarpa.*
**SEQ ID NO: 12** is the amino acid sequence, **SEQ ID NO: 41** is the DNA sequence of POPTRDRAFT_653902 (Accession Nos. XP_002308561 and XM_002308525) from *Populus trichocarpa.*
**SEQ ID NO: 13** is the amino acid sequence, **SEQ ID NO: 42** is the DNA sequence of POPTRDRAFT_664385 (Accession Nos. XP_002320320 and XM_002320284) from *Populus trichocarpa.*
**SEQ ID NO: 14** is the amino acid sequence, **SEQ ID NO: 43** is the DNA sequence of POPTRDRAFT_782963 (Accession Nos. XP_002328578 and XM_002328542) from *Populus trichocarpa.*
**SEQ ID NO: 15** is the amino acid sequence, **SEQ ID NO: 44** is the DNA sequence of POPTRDRAFT_880193 (Accession Nos. XP_002315462 and XM_002315426) from *Populus trichocarpa.*
**SEQ ID NO: 16** is the amino acid sequence, **SEQ ID NO: 45** is the DNA sequence of RCOM_1046780 (Accession Nos. XP_002514107 and XM_002514061) from *Ricinus communis.*
**SEQ ID NO: 17** is the amino acid sequence, **SEQ ID NO: 46** is the DNA sequence of RCOM_0999460 (Accession Nos. XP_002519177 and XM_002519131) from *Ricinus communis.*
**SEQ ID NO: 18** is the amino acid sequence, **SEQ ID NO: 47** is the DNA sequence of RCOM_0797450 (Accession Nos. XP_002516445 and XM_002516339) from *Ricinus communis.*
**SEQ ID NO: 19** is the amino acid sequence, **SEQ ID NO: 48** is the DNA sequence of SORBIDRAFT_01g002270 (Accession Nos. XP_002466151 and XM_002466106) from *Sorghum bicolor.*
**SEQ ID NO: 20** is the amino acid sequence, **SEQ ID NO: 49** is the DNA sequence of SORBIDRAFT_03g047520 (Accession Nos. XP_002459187 and XM_002459142) from *Sorghum bicolor.*
**SEQ ID NO: 21** is the amino acid sequence, **SEQ ID NO: 50** is the DNA sequence of SORBIDRAFT-04g007750 (Accession Nos. XP_002453545 and XM_002453500) from *Sorghum bicolor.*
**SEQ ID NO: 22** is the amino acid sequence, **SEQ ID NO: 51** is the DNA sequence of SORBIDRAFT_10g022580 (Accession Nos. XP_002437185 and XM_002437140) from *Sorghum bicolor.*
**SEQ ID NO: 23** is the amino acid sequence, **SEQ ID NO: 52** is the DNA sequence of LOC100247474 (Accession Nos. XP_002279283 and XM_002279247) from *Vitis vinifera.*
**SEQ ID NO: 24** is the amino acid sequence, **SEQ ID NO: 53** is the DNA sequence of LOC100253375 (Accession Nos. XP_002275498 and XM_002275462) from *Vitis vinifera.*
**SEQ ID NO: 25** is the amino acid sequence, **SEQ ID NO: 54** is the DNA sequence of LOC100256170 (Accession Nos. XP_002278369 and XM_002278333) from *Vitis vinifera.*
**SEQ ID NO: 26** is the amino acid sequence, **SEQ ID NO: 55** is the DNA sequence of LOC100257206 (Accession Nos. XP_002269588 and XM_002269552) from *Vitis vinifera.*
**SEQ ID NO: 27** is the amino acid sequence, **SEQ ID NO: 56** is the DNA sequence of LOC100261004 (Accession Nos. XP_002282173 and XM_002282137) from *Vitis vinifera.*
**SEQ ID NO: 28** is the amino acid sequence, **SEQ ID NO: 57** is the DNA sequence of LOC100191563 (Accession Nos. NP_001130465 and NM_001136993) from *Zea mays.*
**SEQ ID NO: 29** is the amino acid sequence, **SEQ ID NO: 58** is the DNA sequence of LOC100284189 (Accession Nos. NP_001150557 and NM_001157085) from *Zea mays.*

Moreover, reference is made to the figures, wherein
**Figure 1** shows the germination (TER, testa rupture) of the mutants *gnc, gnl* and *gnc gnl* in comparison to the wild-type (Col-O) and to the known GA pathway mutants *spy-3* and *rga-24* gai-t6 (with L*er* as wild-type). Germination (in %) was evaluated 12, 15, 18, 21, 24 and 36 hours after imbibition;
**Figure 2** shows the flowering behaviour of the mutants *gnc, gnl* and *gnc gnl* in comparison to the wild-type (*Col-O*) and to the known GA mutants *spy-*3*, gai-*1 and *rga-*24 *gai-*t6 (with Ler as wild-type). The flowering time (bolting or flowering) can be measured in days or number of leaves formed until inflorescence stem elongation (bolting) or first flower formation. DAG = Days After Germination;
**Figure 3** shows the germination (TER, testa rupture) of seeds from lines overexpressing GNC as a C-terminal GFP fusion protein (GNC-OX) or GNL as an N-terminal YFP fusion (GNL-OX) as compared to that of seed of the wild type (Col-O). Germination (in %) was evaluated 12, 15, 18, 21, 24, 36 and 48 hours after imbibition;
**Figure 4** shows the effect of overexpressing GNC as a C-terminal GFP fusion protein in plants (GNC-GFP #1, 3 and 4) as compared to the wild-type plant (Col-O);
**Figure 5** is an amino acid sequence alignment of polypeptides according to the present invention (SEQ ID NOs: 1-29) showing the conserved GATA DNA-binding domain; and
**Figure 6** is an amino acid sequence alignment of polypeptides according to the present invention (SEQ ID NOs: 1-29) showing the conserved domain located at the C-terminus of these polypeptides.

The invention will now be further described by the following examples, which are given to illustrate the invention, not to limit it.

### EXAMPLES

### Identification of GNC and GNL as DELLA/PIF target genes.

The genes *GNC* and *GNL* have initially been identified as genes whose transcription is reduced in response to GA treatment of the wild-type and also in GA-deficient GA biosynthesis mutants in the plant model species *Arabidopsis thaliana.* The subsequent genetic analyses, initially *of gnc* and *gnl* single and double loss-of function mutants and later of GNC and GNL overexpression lines, revealed that both genes contribute to the repression of seed germination, the repression of flowering and elongation growth. Since *gnc* and *gnl* mutants resemble specific *pif* mutants and since PIF transcription factor activity is regulated by the DELLA proteins of the GA pathway, it was hypothesized that the GA control of *GNC* and *GNL* expression is mediated by PIFs. This hypothesis was confirmed for both genes using chromatin immunoprecipitation of PIF3 from *Arabidopsis thaliana* and by examining the misexpression of these genes in *pif* mutant and *PIF3* overexpression backgrounds. Besides the phenotypic similarities between *gnc* and *gnl* mutants and GA pathway or *pif* mutants, three observations further support the notion that GNC and GNL are important transcription regulators downstream of GA and PIFs: (1) *gnc gnl* mutations can partially suppress the severe phenotype of a *ga1* mutant. Notably, the *gnc* and *gnl* mutations can induce flowering in the *ga1* background; (2) GNC and GNL overexpression lines misexpress about 2000 genes that are also misexpressed in the *ga1* mutant; an additional 1600 genes are misexpressed in *ga1* and in at least the GNC or the GNL overexpression lines; and (3) GNC and GNL overexpression lines misexpress about 1600 genes that are also misexpressed in a *pif1 pif3 pif4 pif5* mutant; additional 500 genes are misexpressed in *pif1 pif3 pif4 pif5* and in at least the GNC or the GNL overexpression lines.

### Inactivation of GNC and/or GNL

Plant lines with inactive *GNC* or *GNL* genes were obtained by screening public resources for so-called T-DNA insertion mutants in said genes. Transgenic seeds were obtained and homozygous progeny was identified by PCR-based genotyping using primers directed against said genes and the T-DNA insert. The loss of *GNC* and *GNL* expression in these lines was confirmed, e.g. in a whole genome transcriptome analysis.

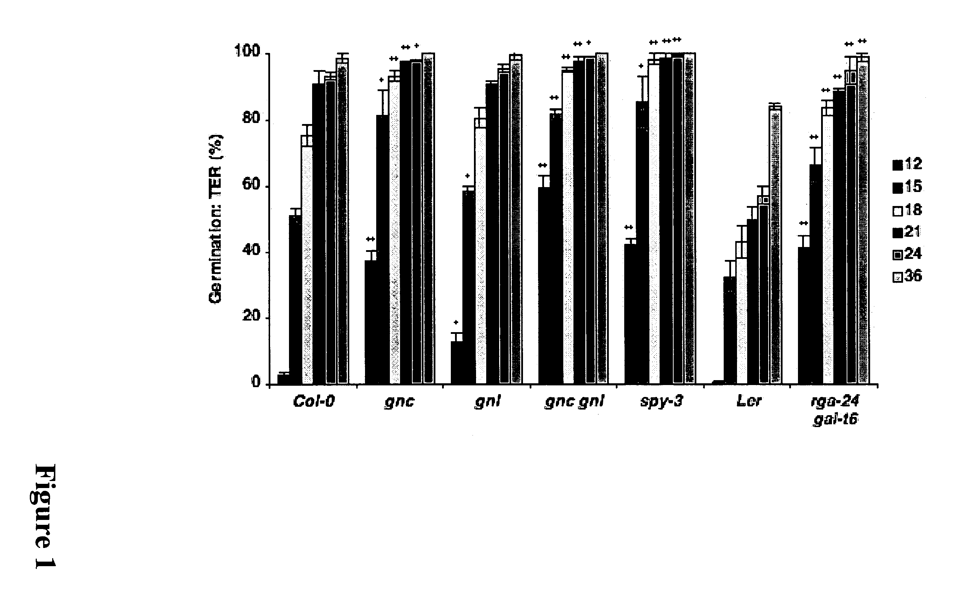

As shown in **Figure 1**, seeds with inactivating mutations *of GNC (gnc)* and *GNL (gnl)* as well as the *gnc gnl* double mutant exhibited an early germination as compared to the wild-type plant (Col-0). The accelerated germination was comparable to that of the known GA pathway mutants *spy-*3 and *rga-*24 *gai-t*6 (with L*er* as wild-type). In the *gnc gnl* double mutant, germination occurred even earlier than in the *gnc* and *gnl* single mutants.

The germination efficiency *of gnc* and *gnl* mutations was determined from after-ripened/non-dormant seeds by monitoring three subsequent steps of germination, namely testa rupture (TER, see **Figure 1**), endosperm rupture and radicle emergence (data not shown) at the time points indicated. At least 80 seeds were scored in each experiment. The experiment shown represents the average of three independent experiments.

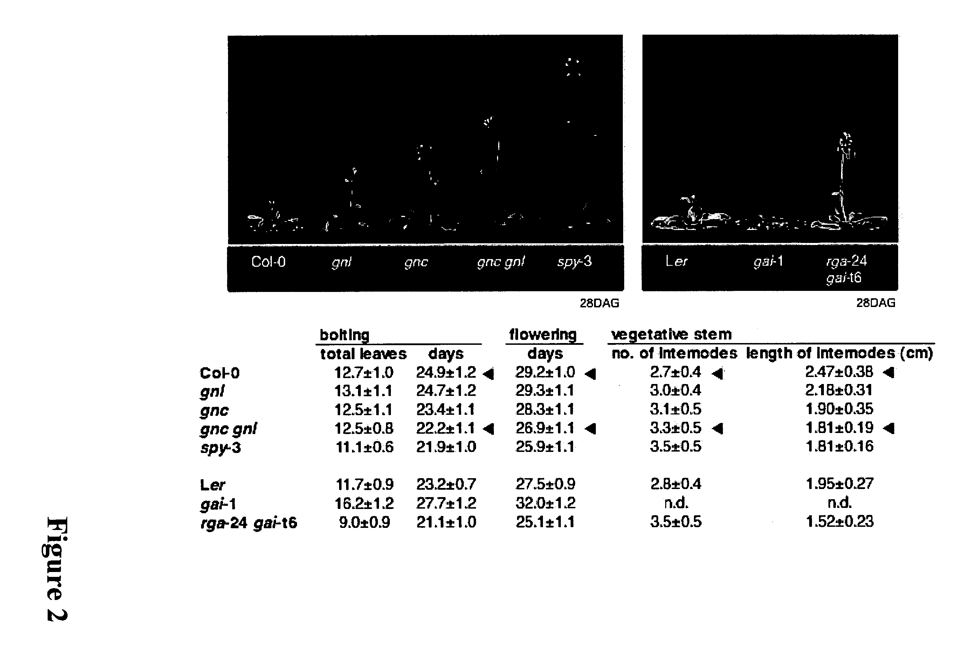

As shown in **Figure 2**, the *gnc* and *gnl* mutants exhibited an early flowering time phenotype as compared to the wild-type plant (Col-0). The effect was further increased in the *gnc gnl* double mutant. The accelerated flowering was comparable to that of the known mutants *spy-3, gai-1* and *rga-*24 *gai-t6* (with L*er* as wild-type). **Figure 2** also shows an increased elongation growth of the mutants as compared to the wild-type plant, particularly in terms of rosette leaf length.

Flowering time was determined from *Arabidopsis thaliana* plants with the genotypes mentioned in the figure. Plants were grown in constant white light (120 µE). The effects observed for the double mutant *gnc gnl* suggest that the two proteins GNC and GNL have redundant function.

### Overexpression of GNC and GNL

To generate the overexpression lines, the GNC and GNL coding sequences were fused in frame with GREEN FLUORESCENT PROTEIN (GFP) or YELLOW FLUORESCENT PROTEIN (YFP) in overexpression vectors. Following plant transformation, transgenic plants were selected and grown in constant light. Microscopic analysis confirmed the predicted nuclear localization of the proteins in transgenic seedlings.

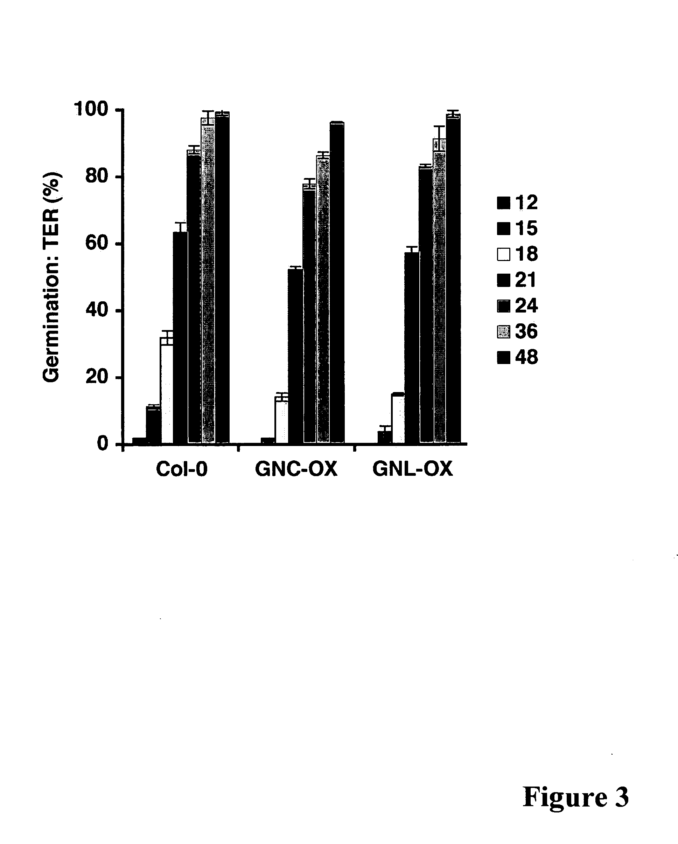

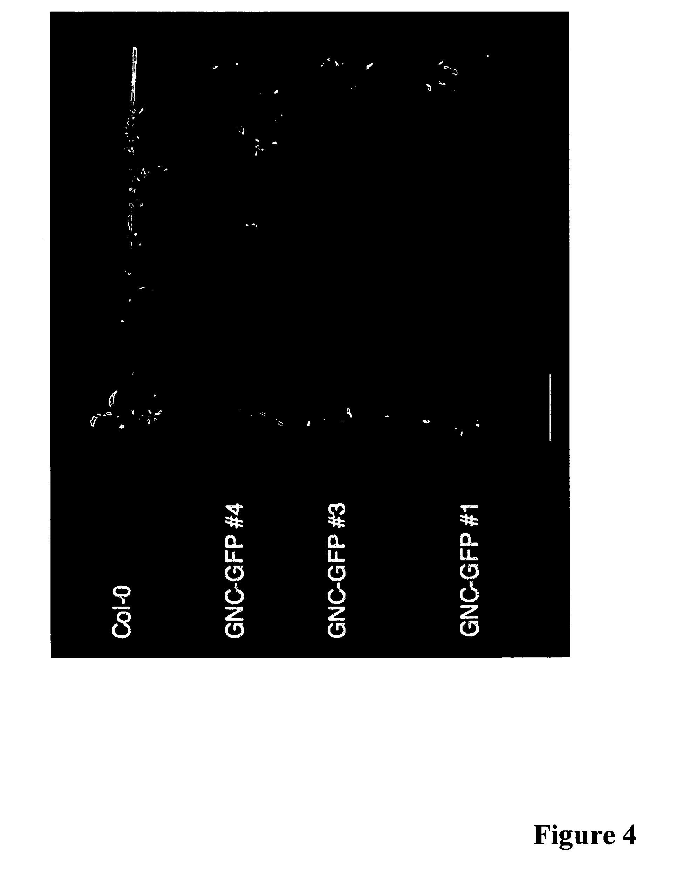

In contrast to the *gnc, gnl* and *gnc gnl* loss-of-function mutants, plants overexpressing GNC or GNL (as GFP/YFP fusion proteins) exhibited a significant delay of germination (**Figure 3**) as well as delayed flowering time and reduced elongation growth (**Figure 4**) as compared to the wild-type plant (Col-O). Most strikingly, plant GNC-GFP #1 still does not show any signs of flowering, while the wild-type plant is fully matured (**Figure 4**).

The features of the present invention disclosed in the specification, the claims, and/or in the accompanying figures may, both separately and in any combination thereof, be material for realizing the invention in various forms thereof.

### REFERENCES

Peng et al. (1999) Nature. 15;400(6741):256-61. 'Green revolution" genes encode mutant gibberellin response modulators.
Schwechheimer (2008) Curr Opin Plant Biol. 11:9-15. Understanding gibberellic acid signalling - are we there yet?
Schwechheimer and Willige (2009) Curr Opin Plant Biol. 12:57-62. Shedding light on gibberellic acid signalling.
Slade and Knauf (2005) Transgenic Res. 14(2):109-15. TILLING moves beyond functional genomics into crop improvement.
Tani et al. (2004) Funct Integr Genomics. 4(4):258-66. Activation tagging in plants: a tool for gene discovery.
Willige et al. (2007) Plant Cell. 19(4):1209-20. The DELLA domain of GA INSENSITIVE mediates the interaction with the GA INSENSITIVE DWARF1A gibberellin receptor of Arabidopsis.

## Claims

1. A method for modulating at least one plant developmental process selected from germination, elongation growth and flowering time, said method comprising:
modulating the activity of at least one polypeptide in a plant or in a plant cell, said polypeptide comprising
a GATA DNA-binding domain and
a conserved domain comprising the amino acid sequence
A — A — Xₙₒₙ₋ₚₒₗₐᵣ Or X_{uncharged polar} ― L ― L ― M ― X ― L ― S ,
wherein Xₙₒₙ₋ₚₒₗₐᵣ is any amino acid having a non-polar side chain, X_{uncharged} polar is any amino acid having an uncharged polar side chain and X is any amino acid.

2. The method according to claim 1, wherein said polypeptide
(i) has an amino acid sequence which is SEQ ID NO: 1 or SEQ ID NO: 2 or a fragment thereof;
(ii) is a homologue to SEQ ID NO: 1 or SEQ ID NO: 2, wherein, preferably, said homologue has an amino acid sequence being selected from SEQ ID NO: 3-29; or
(iii) has an amino acid sequence which is at least 50%, preferably at least 60%, more preferably at least 70%, even more preferably at least 80%, even more preferably at least 90%, and most preferably at least 95% identical to any of SEQ ID NO: 1-29 or a fragment thereof.

3. The method according to claim 1 or 2, wherein said modulating the activity of said polypeptide results in a decreased activity of said polypeptide.

4. The method according to claim 3, wherein said modulating the activity of said polypeptide occurs by one of the following:
(i) producing a mutant nucleic acid coding for a version of said polypeptide having decreased activity, preferably a non-functional version of said polypeptide, through insertion, deletion or mutation of at least one nucleotide in the nucleic acid coding for said polypeptide, and introducing said mutant nucleic acid into a plant cell;
(ii) producing a mutant nucleic acid coding for a version of said polypeptide having reduced expression, through insertion, deletion, mutation of at least one nucleotide at a position 1-500 nucleotides, preferably 1-100 nucleotides upstream of the transcription start site of the nucleic acid coding for said polypeptide, and introducing said mutant nucleic acid into a plant cell;
(iii) producing a mutant nucleic acid by placing the nucleic acid coding for said polypeptide under the control of a regulatory sequence that switches off the expression of said nucleic acid coding for said polypeptide, and introducing said mutant nucleic acid into a plant cell;
(iv) producing a nucleic acid coding for a polypeptide that represses the transcription of the nucleic acid coding for said polypeptide, preferably by binding to the upstream regulatory region of the nucleic acid coding for said polypeptide, and introducing said nucleic acid into a plant cell;
(v) producing, within a plant cell, a mutant nucleic acid coding for a version of said polypeptide having decreased activity, preferably a non-functional version of said polypeptide, or a version of said polypeptide having reduced expression, by exposing said plant cell to a mutagenic substance, such as ethyl methane sulfonate, or by exposing said plant cell to mutagenic radiation, such as UV-radiation or X-rays, and identifying the resultant mutant nucleic acid by gene sequencing, by the recognition of mismatch hybridizations by TILLING, or by related techniques;
(vi) introducing small regulatory RNAs directed at the nucleic acid coding for said polypeptide or at its transcript into a plant cell;
(vii) introducing antisense nucleic acid molecules complementary to the nucleic acid coding for said polypeptide into a plant cell;
(viii) introducing sense nucleic acid molecules homologous to the nucleic acid coding for said polypeptide into a plant cell; or
(ix) expressing a dominant-negative fragment of said polypeptide in a plant cell.

5. The method according to claim 1 or 2, wherein said modulating the activity of said polypeptide results in an increased activity of said polypeptide.

6. The method according to claim 5, wherein said modulating the activity of said polypeptide occurs by one of the following:
(i) producing a mutant nucleic acid coding for a version of said polypeptide having increased activity or a version of said polypeptide having increased expression, through insertion, deletion, mutation of at least one nucleotide in the nucleic acid coding for said polypeptide or at a position 1-500 nucleotides, preferably 1-100 nucleotides upstream of the transcription start site of the nucleic acid coding for said polypeptide, and introducing said mutant nucleic acid into a plant cell;
(ii) producing a mutant nucleic acid, within a plant cell, by placing the nucleic acid coding for said polypeptide under the control of a regulatory sequence that increases expression of said nucleic acid coding for said polypeptide;
(iii) producing, within a plant cell, a mutant nucleic acid coding for a version of said polypeptide having increased activity or a version of said polypeptide having increased expression, by exposing said plant cell to a mutagenic substance, such as ethyl methane sulfonate, or by exposing said plant cell to mutagenic radiation, such as UV-radiation or X-rays, and identifying the resultant mutant nucleic acid by gene sequencing, by the recognition of mismatch hybridizations by TILLING, or by related techniques;
(iv) misexpressing the nucleic acid coding for said polypeptide in a plant cell;
(v) overexpressing said polypeptide in a plant cell;
(vi) producing a nucleic acid coding for a polypeptide that increases the transcription of the nucleic acid coding for said polypeptide, preferably by binding to the upstream regulatory region of the nucleic acid coding for said polypeptide, and introducing said nucleic acid into a plant cell; or
(vii) producing a mutant nucleic acid coding for a version of said polypeptide having increased protein stability.

7. The method according to claim 4 or 6, wherein said nucleic acid coding for said polypeptide has a nucleic acid sequence which is
(i) any of SEQ ID NO: 30-58 or a fragment thereof; or
(ii) at least 50%, preferably at least 60%, more preferably at least 70%, even more preferably at least 80%, even more preferably at least 90%, and most preferably at least 95% identical to any of SEQ ID NO: 30-58 or a fragment thereof.

8. A mutant nucleic acid of a nucleic acid coding for a polypeptide as defined in claim 1 or 2, the expression of which mutant nucleic acid in a plant or in a plant cell results in less activity or more activity of said polypeptide.

9. An overexpression vector comprising a nucleic acid coding for a polypeptide as defined in claim 1 or 2.

10. The mutant nucleic acid or overexpression vector according to claim 8 or 9, wherein said nucleic acid coding for said polypeptide has a nucleic acid sequence which is
(i) any of SEQ ID NO: 30-58 or a fragment thereof; or
(ii) at least 50%, preferably at least 60%, more preferably at least 70%, even more preferably at least 80%, even more preferably at least 90%, and most preferably at least 95% identical to any of SEQ ID NO: 30-58 or a fragment thereof.

11. A nucleic acid consisting of 15 to 21 or 21 to 50, or 50 to 100, or 100 to 150, or 150 to 200 contiguous nucleotides, preferably ribonucleotides, of the nucleic acid coding for a polypeptide as defined in claim 1 or 2.

12. The nucleic acid according to claim 11, wherein said nucleic acid coding for said polypeptide has a nucleic acid sequence which is
(i) any of SEQ ID NO: 30-58 or a fragment thereof; or
(ii) at least 50%, preferably at least 60%, more preferably at least 70%, even more preferably at least 80%, even more preferably at least 90%, and most preferably at least 95% identical to any of SEQ ID NO: 30-58 or a fragment thereof.

13. A plant cell comprising a nucleic acid as defined in any of claims 8 to 12.

14. A plant comprising one or several plant cells according to claim 13.

15. A plant **characterized by** the modulation of at least one developmental process selected from germination, elongation growth and flowering time, obtained by the method according to any of claims 1 to 7.


SEQUENCE LISTING

<110>  Technische Universität München

<120>  Modulation of germination, elongation growth and flowering time
       in plants

<130>  FB23120

<160>  58

<170>  PatentIn version 3.5

<210>  1
<211>  398
<212>  PRT
<213>  Arabidopsis thaliana

<400>  1

Met Asp Ser Asn Phe His Tyr Ser Ile Asp Leu Asn Glu Asp Gln Asn
1               5                   10                  15

His His Glu Gln Pro Phe Phe Tyr Pro Leu Gly Ser Ser Ser Ser Leu
            20                  25                  30

His His His His His His His His His Gln Val Pro Ser Asn Ser Ser
        35                  40                  45

Ser Ser Ser Ser Ser Ile Ser Ser Leu Ser Ser Tyr Leu Pro Phe Leu
    50                  55                  60

Ile Asn Ser Gln Glu Asp Gln His Val Ala Tyr Asn Asn Thr Tyr His
65                  70                  75                  80

Ala Asp His Leu His Leu Ser Gln Pro Leu Lys Ala Lys Met Phe Val
                85                  90                  95

Ala Asn Gly Gly Ser Ser Ala Cys Asp His Met Val Pro Lys Lys Glu
            100                 105                 110

Thr Arg Leu Lys Leu Thr Ile Arg Lys Lys Asp His Glu Asp Gln Pro
        115                 120                 125

His Pro Leu His Gln Asn Pro Thr Lys Pro Asp Ser Asp Ser Asp Lys
    130                 135                 140

Trp Leu Met Ser Pro Lys Met Arg Leu Ile Lys Lys Thr Ile Thr Asn
145                 150                 155                 160

Asn Lys Gln Leu Ile Asp Gln Thr Asn Asn Asn His Lys Glu Ser
                165                 170                 175

Asp His Tyr Pro Leu Asn His Lys Thr Asn Phe Asp Glu Asp His His
            180                 185                 190

Glu Asp Leu Asn Phe Lys Asn Val Leu Thr Arg Lys Thr Thr Ala Ala
        195                 200                 205

Thr Thr Glu Asn Arg Tyr Asn Thr Ile Asn Glu Asn Gly Tyr Ser Asn
    210                 215                 220

Asn Asn Gly Val Ile Arg Val Cys Ser Asp Cys Asn Thr Thr Lys Thr
225                 230                 235                 240

Pro Leu Trp Arg Ser Gly Pro Arg Gly Pro Lys Ser Leu Cys Asn Ala
                245                 250                 255

Cys Gly Ile Arg Gln Arg Lys Ala Arg Arg Ala Ala Met Ala Ala Ala
            260                 265                 270

Ala Ala Ala Gly Asp Gln Glu Val Ala Val Ala Pro Arg Val Gln Gln
        275                 280                 285

Leu Pro Leu Lys Lys Lys Leu Gln Asn Lys Lys Lys Arg Ser Asn Gly
    290                 295                 300

Gly Glu Lys Tyr Asn His Ser Pro Pro Met Val Ala Lys Ala Lys Lys
305                 310                 315                 320

Cys Lys Ile Lys Glu Glu Glu Glu Lys Glu Met Glu Ala Glu Thr Val
                325                 330                 335

Ala Gly Asp Ser Glu Ile Ser Lys Ser Thr Thr Ser Ser Asn Ser Ser
            340                 345                 350

Ile Ser Ser Asn Lys Phe Cys Phe Asp Asp Leu Thr Ile Met Leu Ser
        355                 360                 365

Lys Ser Ser Ala Tyr Gln Gln Val Phe Pro Gln Asp Glu Lys Glu Ala
    370                 375                 380

Ala Val Leu Leu Met Ala Leu Ser Tyr Gly Met Val His Gly
385                 390                 395

<210>  2
<211>  352
<212>  PRT
<213>  Arabidopsis thaliana

<400>  2

Met Gly Ser Asn Phe His Tyr Thr Ile Asp Leu Asn Glu Asp Gln Asn
1               5                   10                  15

His Gln Pro Phe Phe Ala Ser Leu Gly Ser Ser Leu His His His Leu
            20                  25                  30

Gln Gln Gln Gln Gln Gln Gln Gln His Phe His His Gln Ala Ser Ser
        35                  40                  45

Asn Pro Ser Ser Leu Met Ser Pro Ser Leu Ser Tyr Phe Pro Phe Leu
    50                  55                  60

Ile Asn Ser Arg Gln Asp Gln Val Tyr Val Gly Tyr Asn Asn Asn Thr
65                  70                  75                  80

Phe His Asp Val Leu Asp Thr His Ile Ser Gln Pro Leu Glu Thr Lys
                85                  90                  95

Asn Phe Val Ser Asp Gly Gly Ser Ser Ser Ser Asp Gln Met Val Pro
            100                 105                 110

Lys Lys Glu Thr Arg Leu Lys Leu Thr Ile Lys Lys Lys Asp Asn His
        115                 120                 125

Gln Asp Gln Thr Asp Leu Pro Gln Ser Pro Ile Lys Asp Met Thr Gly
    130                 135                 140

Thr Asn Ser Leu Lys Trp Ile Ser Ser Lys Val Arg Leu Met Lys Lys
145                 150                 155                 160

Lys Lys Ala Ile Ile Thr Thr Ser Asp Ser Ser Lys Gln His Thr Asn
                165                 170                 175

Asn Asp Gln Ser Ser Asn Leu Ser Asn Ser Glu Arg Gln Asn Gly Tyr
            180                 185                 190

Asn Asn Asp Cys Val Ile Arg Ile Cys Ser Asp Cys Asn Thr Thr Lys
        195                 200                 205

Thr Pro Leu Trp Arg Ser Gly Pro Arg Gly Pro Lys Ser Leu Cys Asn
    210                 215                 220

Ala Cys Gly Ile Arg Gln Arg Lys Ala Arg Arg Ala Ala Met Ala Thr
225                 230                 235                 240

Ala Thr Ala Thr Ala Val Ser Gly Val Ser Pro Pro Val Met Lys Lys
                245                 250                 255

Lys Met Gln Asn Lys Asn Lys Ile Ser Asn Gly Val Tyr Lys Ile Leu
            260                 265                 270

Ser Pro Leu Pro Leu Lys Val Asn Thr Cys Lys Arg Met Ile Thr Leu
        275                 280                 285

Glu Glu Thr Ala Leu Ala Glu Asp Leu Glu Thr Gln Ser Asn Ser Thr
    290                 295                 300

Met Leu Ser Ser Ser Asp Asn Ile Tyr Phe Asp Asp Leu Ala Leu Leu
305                 310                 315                 320

Leu Ser Lys Ser Ser Ala Tyr Gln Gln Val Phe Pro Gln Asp Glu Lys
                325                 330                 335

Glu Ala Ala Ile Leu Leu Met Ala Leu Ser His Gly Met Val His Gly
            340                 345                 350

<210> 3
<211> 162
<212> PRT
<213> Hordeum vulgare

<400> 3

Met Ser Ser Ser Val Glu Lys Gly Ser Gly Ser Leu Asp Pro Gly Glu
1               5                   10                  15

Arg Pro Ala Ser Gly Ser Gln Pro Lys Ala Cys Thr Ala Cys Asn Thr
            20                  25                  30

Thr Lys Thr Pro Leu Trp Arg Gly Gly Pro Ser Gly Pro Met Ser Leu
        35                  40                  45

Cys Asn Ala Cys Gly Ile Arg Tyr Arg Lys Lys Arg Arg Glu Ala Leu
    50                  55                  60

Gly Leu Asp Glu Pro Pro Lys Lys Arg Gln Pro Ala Ala Ala Ala Ser
65                  70                  75                  80

Ala Ala Ala Ala Ala Ala Cys Ser Glu Ala Gly Gly Glu Ser Ala Glu
                85                  90                  95

Pro Asp Gln Gln Gln Gln Gln Pro Lys Lys Lys Thr Thr Thr Thr Lys
            100                 105                 110

Arg Gly Arg Glu Val Glu Leu Arg Val Val Gly Phe Gly Lys Glu Val
        115                 120                 125

Val Leu Lys Gln Arg Arg Arg Met Arg Arg Arg Arg Arg Leu Gly Glu
    130                 135                 140

Glu Glu Lys Ala Ala Ile Leu Leu Met Ala Leu Ser Ser Gly Val Ile
145                 150                 155                 160

Tyr Gly

<210>  4
<211>  181
<212>  PRT
<213>  Hordeum vulgare

<400>  4

Met Ser Pro Ala Glu Met Gly Ser Asp Lys Met Lys Met Lys Met Lys
1               5                   10                  15

Met Ile Glu Val Glu Val Gln Ala Ala Ala Ala Asp Pro Asp Asp Cys
            20                  25                  30

Thr Ala Ser Gly Ala Gly Asp Pro Lys Ser Cys Ala Asp Cys Asn Thr
        35                  40                  45

Thr Lys Thr Pro Leu Trp Arg Gly Gly Pro Asn Gly Pro Lys Ser Leu
    50                  55                  60

Cys Asn Ala Cys Gly Ile Arg Tyr Arg Lys Arg Arg Arg Val Ala Met
65                  70                  75                  80

Gly Leu Asp Pro Glu Ala Lys Arg Lys Pro Lys Arg Asp Asp Ala Ile
                85                  90                  95

Asn Ser Ala Ala Ala Ala Ala Glu Ala Ser Thr Gln Gln Gln Glu Glu
            100                 105                 110

Val Thr Lys Pro Thr Asp Asp Asp Lys Ala Val Ser Thr Asn Lys Thr
        115                 120                 125

Thr Lys Thr His Thr Val Glu Leu His Met Val Gly Phe Gly Lys Asp
    130                 135                 140

Ala Val Leu Lys Gln Arg Arg Arg Met Arg Arg Arg Lys Pro Ser Cys
145                 150                 155                 160

Leu Gly Glu Glu Glu Arg Ala Ala Met Leu Leu Met Ala Leu Ser Ser
                165                 170                 175

Gly Val Ile Tyr Ala
            180

<210>  5
<211>  203
<212>  PRT
<213>  Nicotiana tabacum

<400>  5

Met Gln Arg Met Lys Asn Ser Asp His Thr His Ile Lys Leu Lys Leu
1               5                   10                  15

Glu Asp Gln Lys Lys Gln Gln Ser Ser Pro Asn Leu Glu Thr Asp Tyr
            20                  25                  30

Ser Ser Asn Ser Ser Ser Asn Asn Ser Asn Asn Asn Asn Ile Pro Ile
        35                  40                  45

Arg Val Cys Ser Asp Cys Asn Thr Thr Lys Thr Pro Leu Trp Arg Ser
    50                  55                  60

Gly Pro Lys Gly Pro Lys Ser Leu Cys Asn Ala Cys Gly Ile Arg Gln
65                  70                  75                  80

Arg Lys Ala Arg Arg Ala Met Ala Ala Ala Ala Ala Ala Ala Ala Asn
                85                  90                  95

Gly Thr Asn Phe Thr Thr Glu Thr Ser Thr Thr Thr Thr Ala Thr Ala
            100                 105                 110

Met Lys Ile Lys Val Gln Gln Lys Gln Lys Ile Thr Thr Lys Val Asn
        115                 120                 125

Asn Asn His Val Val Pro Phe Lys Lys Arg Cys Lys Phe Ile Thr Ser
    130                 135                 140

Thr Thr Ala Ala Val Gly Ser Ser Ser Ser Thr Ile Asn Asn Ala Gln
145                 150                 155                 160

Lys Lys Ile Cys Phe Glu Asp Phe Phe Val Asn Leu Ser Asn Asn Leu
                165                 170                 175

Ala Leu His Arg Val Phe Pro Gln Asp Glu Lys Glu Ala Ala Ile Leu
            180                 185                 190

Leu Met Ala Leu Ser Ser Gly Leu Val His Gly
        195                 200

<210> 6
<211> 353
<212> PRT
<213> Oryza sativa

<400> 6

Met Ser Thr Ile Tyr Met Ser Gln Leu Pro Ala Thr Leu Pro Leu Met
1               5                   10                  15

Glu Gly Asp Gln Asp Gln Gly Leu Tyr Pro Ala Phe His Arg Ala Lys
            20                  25                  30

Asp Pro Pro Ile Leu Phe Pro Phe Met Ile Asp Ser Ala Val Glu His
        35                  40                  45

Gln Gly Gln Ile Tyr Gly Asp Gln Gly Leu Arg Arg Gln Gln Val Leu
    50                  55                  60

Gly Glu Ser Asn Gln Gln Phe Asn Asp His Met Met Met Gly Gly Ser
65                  70                  75                  80

Asp Val Phe Leu Thr Pro Ser Pro Phe Arg Pro Thr Ile Gln Ser Ile
                85                  90                  95

Gly Ser Asp Met Ile Gln Arg Ser Ser Tyr Asp Pro Tyr Asp Ile Glu
            100                 105                 110

Ser Asn Asn Lys Gln His Ala Asn Gly Ser Thr Ser Lys Trp Met Ser
        115                 120                 125

Thr Pro Pro Met Lys Met Arg Ile Ile Arg Lys Gly Ala Ala Thr Asp
    130                 135                 140

Pro Glu Gly Gly Ala Val Arg Lys Pro Arg Arg Arg Ala Gln Ala His
145                 150                 155                 160

Gln Asp Glu Ser Gln Gln Gln Leu Gln Gln Ala Leu Gly Val Val Arg
                165                 170                 175

Val Cys Ser Asp Cys Asn Thr Thr Lys Thr Pro Leu Trp Arg Ser Gly
            180                 185                 190

Pro Cys Gly Pro Lys Ser Leu Cys Asn Ala Cys Gly Ile Arg Gln Arg
        195                 200                 205

Lys Ala Arg Arg Ala Met Ala Ala Ala Ala Asn Gly Gly Ala Ala Val
    210                 215                 220

Ala Pro Ala Lys Ser Val Ala Ala Ala Pro Val Asn Asn Lys Pro Ala
225                 230                 235                 240

Ala Lys Lys Glu Lys Arg Ala Ala Asp Val Asp Arg Ser Leu Pro Phe
                245                 250                 255

Lys Lys Arg Cys Lys Met Val Asp His Val Ala Ala Ala Val Ala Ala
            260                 265                 270

Thr Lys Pro Thr Ala Ala Gly Glu Val Val Ala Ala Ala Pro Lys Asp
        275                 280                 285

Gln Asp His Val Ile Val Val Gly Gly Glu Asn Ala Ala Ala Thr Ser
    290                 295                 300

Met Pro Ala Gln Asn Pro Ile Ser Lys Ala Ala Ala Thr Ala Ala Ala
305                 310                 315                 320

Ala Ala Ala Ser Pro Ala Phe Phe His Gly Leu Pro Arg Asp Glu Ile
                325                 330                 335

Thr Asp Ala Ala Met Leu Leu Met Thr Leu Ser Cys Gly Leu Val His
            340                 345                 350

Ser

<210>  7
<211>  136
<212>  PRT
<213>  Oryza sativa

<400>  7

Met Asp Ser Ser Ser Val Glu Lys Gly Ser Gly Ser Ile Asp Pro Asp
1               5                   10                  15

Glu Arg Thr Ala Ser Gly Glu Pro Lys Ala Cys Thr Asp Cys His Thr
            20                  25                  30

Thr Lys Thr Pro Leu Trp Arg Gly Gly Pro Ser Gly Pro Lys Ser Leu
        35                  40                  45

Cys Asn Ala Cys Gly Ile Arg Tyr Arg Lys Lys Arg Arg Glu Ala Leu
    50                  55                  60

Gly Leu Asp Ala Gly Glu Gly Gly Ala Glu Arg Gln Glu Lys Lys Lys
65                  70                  75                  80

Ser Lys Arg Glu Arg Gly Glu Glu Val Thr Met Glu Leu Arg Met Val
                85                  90                  95

Gly Phe Gly Lys Glu Val Val Leu Lys Gln Arg Arg Arg Met Arg Arg
            100                 105                 110

Arg Arg Arg Leu Gly Glu Glu Glu Lys Ala Ala Ile Leu Leu Met Ala
        115                 120                 125

Leu Ser Ser Gly Val Ile Tyr Ala
    130                 135

<210>  8
<211>  151
<212>  PRT
<213>  Oryza sativa

<400>  8

Met Gly Ser Ser Asp Gln Lys Val Ile Gly Ile Ala Ala Ala Ala Ala
1               5                   10                  15

Ala Ala Ala Glu Glu Ala Gly Arg Arg Cys Cys Val Glu Cys Gly Ala
            20                  25                  30

Thr Thr Thr Pro Met Trp Arg Gly Gly Pro Thr Gly Pro Arg Ser Leu
        35                  40                  45

Cys Asn Ala Cys Gly Ile Arg Tyr Arg Lys Lys Arg Arg Gln Glu Leu
    50                  55                  60

Gly Leu Asp Lys Lys Gln Gln Gln Glu His His Pro His His His Gln
65                  70                  75                  80

Gln Gln Gln Gln Gln Tyr Gln Arg Gln Gln Gln Gln Gln Gln Gln Glu
                85                  90                  95

Asp His Ser Asp Ala Ala Ser Ser Val Lys Asp Ser Ser Ser Ser Ser
            100                 105                 110

Ser Asn Lys Ser Ser Ser Leu Gln Val Val Lys Lys Arg Arg Val Leu
        115                 120                 125

Met Gly Val Glu Glu Ala Ala Ile Leu Leu Met Ala Leu Ser Ser Ser
    130                 135                 140

Ser Pro Thr Leu Leu His Gly
145                 150

<210>  9
<211>  347
<212>  PRT
<213>  Oryza sativa

<400>  9

Met Ser Thr Ile Tyr Met Ser Gln Leu Ser Ala Ala Leu Pro Leu Met
1               5                   10                  15

Glu Gly Glu His His His His His Gln Asp His His Gln Gly His Phe
            20                  25                  30

Gln Ala Phe Ser Leu Gln Pro Lys Asp Pro Pro Val Leu Phe Pro Phe
        35                  40                  45

Val Ile Ser Arg Arg Ser Ser Ser Ser Ser Pro Ser Asp Ser Thr Thr
    50                  55                  60

Leu Ser Tyr Gly Ser Asp His His Leu Thr Gln Gln Gln Gln His Gln
65                  70                  75                  80

His Gln Ala Met Leu Glu Pro Gln Asn Met Ile Gly Gly Ser Ser Ala
                85                  90                  95

Gly Ile Phe Ala Thr Pro Phe Pro Thr Val Lys Ser Ile Arg Asp Asp
            100                 105                 110

Met Ile Glu Arg Ser Gln Phe Asp Pro Tyr Asp Thr Glu Lys Leu Gln
        115                 120                 125

Ala Ser Cys Gly Leu Ala Lys Val Val Ala Gly Gly Lys Trp Ser Ala
    130                 135                 140

Val Pro Ala Ala Lys Met Lys Ile Thr Arg Lys Met Gly Glu Pro Ser
145                 150                 155                 160

Ser Gly Val Thr Gly Gly Ala Ala Thr Thr Val Ala Pro Lys Lys Pro
                165                 170                 175

Arg Arg Arg Pro Ala Gln Ala Tyr Glu Asp His Gly His Gly Gly Ala
            180                 185                 190

Met Gly Gln Ala Phe Gly Val Ile Arg Val Cys Ser Asp Cys Asn Thr
        195                 200                 205

Thr Lys Thr Pro Leu Trp Arg Ser Gly Pro Cys Gly Pro Lys Ser Leu
    210                 215                 220

Cys Asn Ala Cys Gly Ile Arg Gln Arg Lys Ala Arg Arg Ala Met Met
225                 230                 235                 240

Ala Ser Gly Leu Pro Ala Ser Pro Asn Ala Ala Gly Pro Lys Ala Ala
                245                 250                 255

Ala His Ser Gly Ala Thr Asn Ala Ala Ala Ala Ala Ala Met Glu Glu
            260                 265                 270

Thr Ala Glu Ser Ala Thr Val Ala Pro Pro Pro Ala Pro Thr Thr Arg
        275                 280                 285

Gly Gly Thr Leu Val Asp Ser Ile Gly Leu Ser Trp Ser Lys Thr His
    290                 295                 300

Ala Ala Ala Thr Ala Ser Cys Ser Phe Arg Pro Ser Pro Val Ala Pro
305                 310                 315                 320

Gly Phe Ala Ala Ala Val Gln Asp Glu Ile Thr Asp Ala Ala Met Leu
                325                 330                 335

Leu Met Thr Leu Ser Cys Gly Leu Val Arg Ser
            340                 345

<210>  10
<211>  147
<212>  PRT
<213>  Populus trichocarpa x Populus deltoides

<400>  10

Met Ile Lys Asp Leu Arg Thr Lys Gly Pro Glu Ser Glu Asp Met Asp
1               5                   10                  15

Asn Thr His Pro Ser Lys Cys Asn Glu Ile Lys Arg Arg Cys Thr Asp
            20                  25                  30

Cys Gln Thr Thr Arg Thr Pro Cys Trp Arg Gly Gly Pro Ala Gly Pro
        35                  40                  45

Arg Thr Leu Cys Asn Ala Cys Gly Ile Arg Gln Arg Lys Lys Arg Arg
    50                  55                  60

Ala Leu Leu Gly Phe Asp Lys Gly Gly Pro Glu Arg Ser Arg Glu Lys
65                  70                  75                  80

Met Ala Lys Gly Ser Asn Ser Ser Lys Leu Gly Val Ser Leu Asn Leu
                85                  90                  95

Gly Leu Met Gly Phe Lys Arg Asp Gly Met Phe Gln Glu Asp Trp Lys
            100                 105                 110

Ile Lys Leu Gly Glu Glu Glu Gln Ala Ala Ile Leu Leu Met Ala Leu
        115                 120                 125

Ser Cys Gly Ser Val Leu Pro Trp Tyr Lys Lys Asp Glu Gly Lys His
    130                 135                 140

Phe Tyr Pro
145

<210>  11
<211>  124
<212>  PRT
<213>  Populus trichocarpa

<400>  11

Ser Glu Asp Met Asp Asn Thr His Pro Ser Lys Cys Asn Glu Ile Lys
1               5                   10                  15

Arg Arg Cys Thr Asp Cys Gln Thr Thr Arg Thr Pro Cys Trp Arg Gly
            20                  25                  30

Gly Pro Ala Gly Pro Arg Thr Leu Cys Asn Ala Cys Gly Ile Arg Gln
        35                  40                  45

Arg Lys Arg Arg Arg Ala Leu Leu Gly Leu Asp Lys Gly Gly Pro Glu
    50                  55                  60

Arg Ser Arg Glu Lys Met Ala Lys Gly Ser Asn Ser Ser Lys Leu Gly
65                  70                  75                  80

Val Ser Leu Asn Leu Asp Leu Met Gly Phe Lys Arg Asp Gly Met Phe
                85                  90                  95

Gln Glu Asp Trp Lys Arg Lys Leu Gly Glu Glu Glu Gln Ala Ala Ile
            100                 105                 110

Leu Leu Met Ala Leu Ser Cys Gly Ser Val Cys Ala
        115                 120

<210>  12
<211>  234
<212>  PRT
<213>  Populus trichocarpa

<400>  12

Met Tyr Asn Ile Ser His Gly Gly Ser Ser Ser Ser Phe Gln Pro Glu
1               5                   10                  15

Val Asn Asp His Asn Tyr Asn Ser Asn Phe His Asn Leu Ser Ser Ser
            20                  25                  30

Lys Met Glu Asp Gly Ala Glu Glu Ser Gly Glu Ser Ser Val Lys Trp
        35                  40                  45

Met Pro Ser Lys Met Arg Leu Met Gln Lys Met Thr Asn Ser Asn Cys
    50                  55                  60

Ser Glu Thr Asp His Met Pro Met Lys Phe Met Leu Lys Phe His Asn
65                  70                  75                  80

Gln Gln Tyr Gln Asn Asn Glu Ile Asn Ser Ser Ser Asn Ser Asn Ser
                85                  90                  95

Asn Ile Arg Val Cys Ser Asp Cys Asn Thr Thr Ser Thr Pro Leu Trp
            100                 105                 110

Arg Ser Gly Pro Arg Gly Pro Lys Ser Leu Cys Asn Ala Cys Gly Ile
        115                 120                 125

Arg Gln Arg Lys Ala Arg Arg Ala Met Ala Ala Ala Ala Ala Ala Ala
    130                 135                 140

Asn Gly Thr Val Ile Ala Ile Glu Ala Ser Ser Ser Thr Arg Ser Thr
145                 150                 155                 160

Lys Val Asn Asn Lys Val Lys Lys Ser Arg Thr Asn His Val Ser Gln
                165                 170                 175

Asn Lys Lys Leu Ser Lys Pro Pro Glu Ser Ser Leu Gln Ser Gln Lys
            180                 185                 190

Lys Leu Cys Phe Lys Asn Leu Ala Leu Ser Leu Ser Lys Asn Pro Ala
        195                 200                 205

Leu Gln Gln Val Leu Pro His Asp Val Glu Glu Ala Ala Ile Leu Leu
    210                 215                 220

Met Glu Leu Ser Cys Gly Phe Ile His Ser
225                 230

<210>  13
<211>  121

<212>  PRT
<213>  Populus trichocarpa

<400>  13

Met Asp Ser Thr Gln Ser Ser Lys Gly Asn Glu Ile Lys Arg Arg Cys
1               5                   10                  15

Met Asp Cys Gln Thr Thr Arg Thr Pro Cys Trp Arg Gly Gly Pro Ala
            20                  25                  30

Gly Pro Arg Thr Leu Cys Asn Ala Cys Gly Ile Arg Gln Arg Lys Lys
        35                  40                  45

Arg Arg Ala Leu His Gly Ser Asp Lys Gly Gly Ala Glu Arg Ser Lys
    50                  55                  60

Asn Lys Ile Ala Lys Ser Ser Asn Ser Ser Lys Leu Gly Val Ser Leu
65                  70                  75                  80

Lys Leu Asp Leu Met Gly Phe Arg Arg Asp Gly Ile Leu Gln Glu Asp
                85                  90                  95

Trp Lys Arg Lys Leu Gly Glu Glu Glu Gln Ala Ala Ile Leu Leu Met
            100                 105                 110

Ala Leu Ser Cys Gly Leu Val Arg Ala
        115                 120

<210>  14
<211>  125
<212>  PRT
<213>  Populus trichocarpa

<400>  14

Met Ser Ser Asn Ser Gln Glu Thr Glu Ser Pro Leu Lys Lys Thr Cys
1               5                   10                  15

Ala Asp Cys Gly Thr Ser Lys Thr Pro Leu Trp Arg Gly Gly Pro Ala
            20                  25                  30

Gly Pro Lys Ser Leu Cys Asn Ala Cys Gly Ile Arg Ser Arg Lys Lys
        35                  40                  45

Lys Arg Asp Ile Leu Gly Leu Asn Lys Gly Ala Ala Asn Asp Lys Arg
    50                  55                  60

Ala Lys Lys Gly Ser Asn Asn Asn Gly Ser Ser Asn Asn Asn Asn Asn
65                  70                  75                  80

Lys Gln Leu Gly Asp Gly Ser Lys Gln Arg Leu Leu Ala Leu Gly Arg
                85                  90                  95

Glu Val Leu Met Gln Arg Arg Lys Leu Gly Glu Glu Glu Gln Ala Ala
            100                 105                 110

Val Leu Leu Met Ala Leu Ser Tyr Gly Ser Val Tyr Ala
        115                 120                 125

<210>  15
<211>  125
<212>  PRT
<213>  Populus trichocarpa

<400>  15

Met Ser Ser Lys Ser Leu Glu Thr Glu Ser Pro Gln Lys Lys Thr Cys
1               5                   10                  15

Ala Asp Cys Gly Thr Ser Lys Thr Pro Leu Trp Arg Gly Gly Pro Ala
            20                  25                  30

Gly Pro Lys Ser Leu Cys Asn Ala Cys Gly Ile Arg Ser Arg Lys Lys
        35                  40                  45

Lys Arg Asp Ile Leu Gly Leu Asn Lys Gly Gly Ala Ala Ala Asn Asp
    50                  55                  60

Lys Arg Ala Lys Lys Gly Ser Thr Asn Asn Gly Ser Ser Asp Gly Leu
65                  70                  75                  80

Lys Gln Arg Leu Leu Ala Leu Gly Arg Glu Val Leu Val Gln Gly Ser
                85                  90                  95

Thr Val Glu Arg Arg Arg Arg Lys Leu Gly Glu Glu Glu Gln Ala Ala
            100                 105                 110

Val Leu Leu Met Ala Leu Ser Tyr Gly Ser Val Tyr Ala
        115                 120                 125

<210>  16
<211>  312
<212>  PRT
<213>  Ricinus communis

<400>  16

Met Thr Pro Thr Tyr His Ser Ser Phe Pro Pro Phe Thr Ile Asp Leu
1               5                   10                  15

Asn Glu Asp Gln His His His Gln Leu Ile Phe Cys Ser Lys Thr Thr
                20                  25                  30

Thr Glu Asp Ala Ser Ser Ser Ser Ser Ile Ser Tyr Pro Ile Phe Ile
        35                  40                  45

Asn Pro Pro Gln Glu Glu Val Gly Tyr Tyr His Lys Glu Leu Gln Pro
    50                  55                  60

Leu His His Gln Glu Val Asp Asn Ile Tyr Ala Ser His Gly Arg Ser
65                  70                  75                  80

Trp Asp His Arg Ile Ile Lys Asn Glu Asn Glu Asn Gly Gln Glu Leu
                85                  90                  95

Ser Val Cys Lys Lys Glu Asp Lys Ser Thr Ser Ile Glu Asp Gln Arg
                100                 105                 110

Asp Asn Ser Ser Val Lys Trp Met Ser Ser Lys Met Arg Leu Met Arg
        115                 120                 125

Lys Met Met Thr Thr Asp Gln Thr Val Asn Thr Thr Gln His Thr Ser
    130                 135                 140

Ser Met His Lys Leu Glu Asp Lys Glu Lys Ser Arg Ser Leu Pro Leu
145                 150                 155                 160

Gln Asp Asp Tyr Ser Ser Lys Asn Leu Ser Asp Asn Ser Asn Asn Thr
                165                 170                 175

Ile Arg Val Cys Ser Asp Cys Asn Thr Thr Lys Thr Pro Leu Trp Arg
                180                 185                 190

Ser Gly Pro Arg Gly Pro Lys Ser Leu Cys Asn Ala Cys Gly Ile Arg
        195                 200                 205

Gln Arg Lys Ala Arg Arg Ala Leu Ala Ala Ala Gln Ala Ser Ala Asn
    210                 215                 220

Gly Thr Ile Phe Ala Pro Asp Thr Ala Ala Met Lys Thr Asn Lys Val
225                 230                 235                 240

Gln Asn Lys Glu Lys Arg Thr Asn Asn Ser His Leu Pro Phe Lys Lys
                245                 250                 255

Arg Cys Lys Phe Thr Ala Gln Ser Arg Gly Ser Arg Lys Lys Leu Cys
            260                 265                 270

Phe Glu Asp Leu Ser Ser Thr Ile Leu Ser Lys Asn Ser Ala Phe Gln
        275                 280                 285

Gln Leu Phe Pro Gln Asp Glu Lys Glu Ala Ala Ile Leu Leu Met Ala
    290                 295                 300

Leu Ser Tyr Gly Leu Val His Gly
305                 310

<210>  17
<211>  149
<212>  PRT
<213>  Ricinus communis

<400>  17

Met Leu Asp Pro Ser Glu Lys Ile Leu Glu Phe Glu Asp Met Ser Ser
1               5                   10                  15

Lys Ser Ala Glu Gly Glu Asn Gln Gln Lys Lys Ser Cys Ala Asp Cys
            20                  25                  30

Gly Thr Thr Lys Thr Pro Leu Trp Arg Gly Gly Pro Ala Gly Pro Lys
        35                  40                  45

Ser Leu Cys Asn Ala Cys Gly Ile Arg Ser Arg Lys Lys Lys Arg Asp
    50                  55                  60

Ser Leu Gly Leu Asn Arg Ala Ser Ser Asn Pro Asp Lys Lys Ser Arg
65                  70                  75                  80

Lys His Ser Ser Ser Asn Gly Ser Ser Asn Asn His Asn Ser Asn Asn
                85                  90                  95

Ser Asn Arg Leu Gly Asp Gly Leu Lys Gln Arg Leu Leu Ala Leu Gly
            100                 105                 110

Arg Glu Val Leu Met Gln Arg Ser Ser Val Glu Lys Gln Arg Arg Lys
        115                 120                 125

Leu Gly Glu Glu Glu Gln Ala Ala Val Leu Leu Met Ala Leu Ser Tyr
    130                 135                 140

Gly Ser Val Tyr Ala
145

<210>  18
<211>  186
<212>  PRT

<213>  Ricinus communis

<400>  18

Met Pro Ser Lys Met Arg Ile Met Gln Lys Met Met Asn Ser Asn Cys
1               5                   10                  15

Phe Glu Phe Asn Asp Lys Pro Val Lys Phe Thr Val Lys Phe Gln Asp
            20                  25                  30

His Gln Gln Tyr Gln Ala Thr Asn Asn Glu Ile Asn Ser Ser Cys Ser
        35                  40                  45

Asn Gly Asn Asn Asn Ile Arg Val Cys Ser Asp Cys Asn Thr Thr Thr
    50                  55                  60

Thr Pro Leu Trp Arg Ser Gly Pro Arg Gly Pro Lys Ser Leu Cys Asn
65                  70                  75                  80

Ala Cys Gly Ile Arg Gln Arg Lys Ala Arg Arg Ala Met Ala Ala Ala
                85                  90                  95

Ala Ala Ile Ala Met Glu Thr Ser Ser Thr Lys Ala Ala Lys Val Lys
            100                 105                 110

Glu Lys Lys Ser Arg Thr Gly His Ala Ser Gln Cys Lys Lys Leu Cys
        115                 120                 125

Lys Pro Pro Asp His Pro Pro Pro Pro Tyr Asn Gln Gly Gln Lys Pro
    130                 135                 140

Lys Val Ser Phe Lys Asn Leu Ala Leu Ser Leu Ser Asn Asn Ser Ala
145                 150                 155                 160

Leu Gln Arg Val Phe Pro Glu Asp Val Glu Glu Ala Ala Thr Leu Leu
                165                 170                 175

Met Glu Leu Ser Cys Gly Phe Ile His Gly
            180                 185

<210>  19
<211>  157
<212>  PRT
<213>  Sorghum bicolor

<400>  19

Met Asp Ser Ser Ala Glu Lys Gln Gly Ser Gly Thr Leu Asp Pro Asp
1               5                   10                  15

Glu Arg Pro Pro Ala Ser Gly Glu Thr Lys Ala Cys Thr Glu Cys His
            20                  25                  30

Thr Thr Lys Thr Pro Leu Trp Arg Gly Gly Pro Cys Gly Pro Met Ser
        35                  40                  45

Leu Cys Asn Ala Cys Gly Ile Arg Tyr Arg Lys Lys Arg Arg Glu Ala
    50                  55                  60

Met Gly Leu Asp Ser Ser Ser Lys Ala Gly Gly Gly Thr Glu Gln Gln
65                  70                  75                  80

Gln Gln Arg Lys Lys Lys Ala Thr Ala Ala Ala Ala Ala Ala Ala Ala
                85                  90                  95

Ser Lys Arg Glu Arg Glu Arg Ser Lys Glu Ala Asp Glu Val Thr Val
            100                 105                 110

Glu Leu Arg Ala Val Gly Phe Gly Lys Glu Val Val Leu Lys Gln Arg
        115                 120                 125

Arg Arg Met Arg Arg Arg Arg Arg Leu Gly Glu Glu Glu Arg Ala Ala
    130                 135                 140

Phe Leu Leu Met Ala Leu Ser Ser Gly Val Val Tyr Ala
145                 150                 155

<210>  20
<211>  217
<212>  PRT
<213>  Sorghum bicolor

<400>  20

Met Glu Val Glu Met Glu Val Glu Met Gly Thr Asp Pro Asn Pro Asn
1               5                   10                  15

Pro Asn Pro Asn Pro Asn Thr Ser Pro Ser Ala Ala Asp Asp Ala Ala
            20                  25                  30

Thr Ala Ser Gly Glu Ala Lys Ala Cys Ala Asp Cys His Thr Thr Lys
        35                  40                  45

Thr Pro Leu Trp Arg Gly Gly Pro Glu Gly Pro Lys Ser Leu Cys Asn
    50                  55                  60

Ala Cys Gly Ile Arg Tyr Arg Lys Arg Arg Arg Gln Ala Leu Gly Leu
65                  70                  75                  80

Asp Ala Ala Ala Ala Asp Ser Gln Gln Asp Gln Gln Gln Pro Lys Lys
                85                  90                  95

Lys Ala Thr Ala Asp Pro Gln Gln Gln Asp Gln His His Leu Arg Lys
            100                 105                 110

Lys Ala Ala Asp Pro Gln Gln Gln Asp Gln His His Leu Arg Lys Lys
        115                 120                 125

Ala Ala Ala Ala Ala Thr Thr Asp Pro Gln Gln Gln Asp Arg Lys Lys
    130                 135                 140

Ala Ala Ala Ala Ser Ser Thr Asn Lys Lys Asp Lys Asp Lys Glu Asp
145                 150                 155                 160

Asn Lys Lys Lys Lys Asp Gln Gln Val Thr Val Glu Leu Arg Val Val
                165                 170                 175

Gly Phe Gly Lys Glu Val Met Leu Lys Gln Arg Arg Arg Met Arg Arg
            180                 185                 190

Lys Lys Cys Met Ser Glu Glu Glu Arg Ala Ala Val Leu Leu Met Ala
        195                 200                 205

Leu Ser Ser Gly Val Ile Tyr Ala Ser
    210                 215

<210>  21
<211>  371
<212>  PRT
<213>  Sorghum bicolor

<400>  21

Met Glu Gly Glu Gln Asp Gln Asp Gln Gly Leu Phe Arg Pro Ala Pro
1               5                   10                  15

Phe His Val Ala Lys Asp Pro Pro Ile Leu Phe Pro Phe Met Ile Thr
            20                  25                  30

Asn Pro Val Asp Gln Leu Gln Gly Gln Ser Ser Ser Tyr Gly Asp Gln
        35                  40                  45

His Leu Arg Gln His Val Leu Ser Glu Ser Thr His Gln Gln Phe Thr
    50                  55                  60

Asp Arg Met Met Met Ser Gly Ser Glu Val Phe Pro Thr Met Pro Ser
65                  70                  75                  80

Pro Phe Arg Gln Thr Ile Gln Ser Ile Asp Gly Asp Met Ile Gln Arg
                85                  90                  95

Ser Ala Tyr Asp Pro Tyr Asp Thr Glu Asn Asn Lys His Thr Glu Gly
            100                 105                 110

Leu Arg Ser Gly Gly Gly Gly Gly Gly Trp Thr Val Ser Ser Ser Pro
        115                 120                 125

Pro Ala Lys Met Lys Ile Met Arg Arg Lys Ala Thr Ser Glu Tyr Pro
    130                 135                 140

Glu Gly Gly Gly Gly Gly Ala Arg Lys Pro Arg Arg Arg Pro Gln Ala
145                 150                 155                 160

His His Gln Asp Ala Glu Ser Gln Leu Leu Gln Gln Gln Gln Gln Ala
                165                 170                 175

Met Gly Gly Val Val Arg Val Cys Ser Asp Cys Asn Thr Thr Lys Thr
            180                 185                 190

Pro Leu Trp Arg Ser Gly Pro Cys Gly Pro Lys Ser Leu Cys Asn Ala
        195                 200                 205

Cys Gly Ile Arg Gln Arg Lys Ala Arg Arg Ala Met Ala Ala Ala Ala
    210                 215                 220

Ala Ala Ala Ala Ala Ser Ser Asn Gly Gly Ala Pro Gln Ala Ala Thr
225                 230                 235                 240

Glu Gln Gln Pro Lys Pro Ala Lys Lys Glu Lys Arg Ser Asp Ile Val
                245                 250                 255

Asp Arg Ser Ser Leu Pro Phe Lys Lys Arg Cys Lys Gln Thr Met Val
            260                 265                 270

Val Val Asp His Ala Ala Gly Val Ala Ala Thr Glu Ala Thr Pro Pro
        275                 280                 285

Pro Pro Pro Pro Glu Thr Ala Ala Leu Thr Lys Asp Gln Ala Asp Val
    290                 295                 300

Asp His Val Ser Ser Asp Lys Lys Gln Val Asn Gly His Gly Ala Thr
305                 310                 315                 320

Ser Leu Glu Ser Ser Lys Ile Val Ala Thr Pro Pro Pro Gln Ala Met
                325                 330                 335

Ser Ser Phe His Ala Phe Pro Ala Asp Glu Ile Thr Asp Ala Ala Met
            340                 345                 350

Leu Leu Met Thr Leu Ser Cys Gly His Ala Arg Leu Gln Leu Ala Met
        355                 360                 365

Ala Ser Ser
    370

<210>  22
<211>  386
<212>  PRT
<213>  Sorghum bicolor

<400>  22

Met Ser Ala Ile Tyr Met Ser Gln Leu Ser Thr Ala Leu Pro Leu Met
1               5                   10                  15

Glu Gly Asp His His His His His His His His His Gln Gly His Phe
            20                  25                  30

Gln Ala Phe Thr Leu Pro Lys Asp Pro Pro Ile Leu Phe Pro Phe Val
        35                  40                  45

Ile Ser Asn Ser Ser Ala Ser Glu Ser Ser Leu Ser Tyr Gly Ser Ala
    50                  55                  60

Asp His His Leu Leu Arg Gln His Arg Gln Thr Met Leu Glu Pro Gln
65                  70                  75                  80

His Met Ile Gly Gly Ser Ser Ser Ala Ala Ser Ser Val Phe Ala Thr
                85                  90                  95

Pro Phe Pro Thr Val Glu Ser Ile Arg Asp Asp Met Ile Glu Pro Ala
            100                 105                 110

Ser Tyr Asp Pro Tyr Asp Met Gly Lys Leu His Gln Val Val Gly Gly
        115                 120                 125

Gly Ser Ser Met Asp Ala Cys Ser Trp Thr Pro Pro Ala Ala Lys Met
    130                 135                 140

Arg Ile Thr Arg Lys Ala Thr Ala Ala Asp Pro Ser Gly Ala Gly Lys
145                 150                 155                 160

Lys Pro Arg Arg Arg Ala His Gln Ala Ala Gly Tyr Asp Ala Asp Ile
                165                 170                 175

Asn Met Ser Gly Gln Pro Asn Leu Gly Val Ile Arg Val Cys Ser Asp
            180                 185                 190

Cys Asn Thr Thr Lys Thr Pro Leu Trp Arg Ser Gly Pro Cys Gly Pro
        195                 200                 205

Lys Ser Leu Cys Asn Ala Cys Gly Ile Arg Gln Arg Lys Ala Arg Arg
    210                 215                 220

Ala Met Met Ala Ala Ala Ser Thr Ser Gly Pro Ala Ala Val Pro Ala
225                 230                 235                 240

Thr Asp Ser Asp Lys Ala Ser Pro Ser Asn Ala Ala Gly Ala Ala Ala
                245                 250                 255

Ala His Pro Lys Val Lys Lys Glu Lys Arg Ser Val Asp Val Asp Arg
            260                 265                 270

Ser Leu Pro Phe Lys Lys Arg Cys Lys Val Val Gln Val Gln Gln Asp
        275                 280                 285

His Ala Ala Ala Val Ala Ala Pro Ala Ala Ala Thr Asp Arg Pro Ala
    290                 295                 300

Val Val Val Gln Gln Ala Ala Thr Ala Ala Glu Val Gly Asp Asp Asp
305                 310                 315                 320

Ala Cys Pro Ser Arg Asp Leu Leu Val Asp Asp Ile Gly Gly Leu Ile
                325                 330                 335

Ser Trp Ser Arg Ser Pro Pro Ala Ala Pro Ala Ser Ala Asp Ala Ala
            340                 345                 350

Ser Cys Ser Phe Arg Ala Ser Pro Ala Leu Pro Val Gln Gln Asp Glu
        355                 360                 365

Ile Thr Asp Ala Ala Met Leu Leu Met Thr Leu Ser Cys Gly Leu Val
    370                 375                 380

Arg Ser
385

<210>  23
<211>  306
<212>  PRT
<213>  Vitis vinifera

<400>  23

Met Thr Pro Val Phe Leu Asn Thr Ser Ser Ser Ser Pro Phe Pro Ala
1               5                   10                  15

Leu Glu Leu Lys Glu Asp His Gln His Phe Gln Leu Leu Phe Ser Thr
            20                  25                  30

Asn Pro Pro Ser Tyr Gln Ala Ser Ser Ser His Pro Cys Pro Ser Phe
        35                  40                  45

Phe Asn Ser Ser Thr Gln Ser Gln Arg Gly Asp His Ser Pro Arg Asp
    50                  55                  60

Pro Gln Gln His Glu Asp Lys Asp Asp Lys Tyr Ile Ser His Gly Gly
65                  70                  75                  80

Cys Gly Glu Ser Gln Val Phe Ser Ser Ser Ser Leu Leu Gln Pro Met
                85                  90                  95

Ala Asp Asp Asn Lys Ser Ser His Lys Leu Ser Val Phe Lys Lys Glu
            100                 105                 110

Glu Gly Asp Glu Gly Asn Lys Ser Thr Glu Lys Trp Met Ser Ser Lys
        115                 120                 125

Met Arg Leu Met Arg Lys Met Met Asn Ser Asp Cys Thr Thr Ala Lys
    130                 135                 140

Ile Glu Gln Lys Val Glu Asp His Gln Gln Trp Asp Asn Ile Asn Glu
145                 150                 155                 160

Phe Asn Ser Ser Asn Asn Thr Ser Asn Ile Pro Ile Arg Val Cys Ser
                165                 170                 175

Asp Cys Asn Thr Thr Lys Thr Pro Leu Trp Arg Ser Gly Pro Arg Gly
            180                 185                 190

Pro Lys Ser Leu Cys Asn Ala Cys Gly Ile Arg Gln Arg Lys Ala Arg
        195                 200                 205

Arg Ala Met Ala Ala Ala Ala Ala Ala Ala Ala Asn Gly Thr Ala Val
    210                 215                 220

Gly Thr Glu Ile Ser Pro Met Lys Met Lys Leu Pro Asn Lys Glu Lys
225                 230                 235                 240

Lys Met His Thr Ser Asn Val Gly Gln Gln Lys Lys Leu Cys Lys Pro
                245                 250                 255

Pro Cys Pro Pro Pro Thr Glu Lys Lys Leu Cys Phe Glu Asp Phe Thr
            260                 265                 270

Ser Ser Ile Cys Lys Asn Ser Gly Phe Arg Arg Val Phe Pro Arg Asp
        275                 280                 285

Glu Glu Glu Ala Ala Ile Leu Leu Met Ala Leu Ser Cys Asp Leu Val
    290                 295                 300

Tyr Ser
305

<210>  24
<211>  153
<212>  PRT
<213>  Vitis vinifera

<400>  24

Met Val Asp Leu Ser Glu Lys Gly Ser Glu Ser Glu Asp Met Asn Asn
1               5                   10                  15

Lys Asn Pro Asp Ala Val Ser Ser Ala Glu Ser Gln Val Asn Glu Pro
            20                  25                  30

Lys Lys Thr Cys Ala Asp Cys Gly Thr Thr Lys Thr Pro Leu Trp Arg
        35                  40                  45

Gly Gly Pro Ala Gly Pro Lys Ser Leu Cys Asn Ala Cys Gly Ile Arg
    50                  55                  60

Ser Arg Lys Lys Arg Arg Ala Phe Leu Gly Leu Asn Lys Gly Ser Thr
65                  70                  75                  80

Asp Asp Arg Lys Ala Lys Arg Ser Ser Asn His Ser His Asn Asn Gly
                85                  90                  95

Gly Gly Asn Gly Asn Asn Lys Leu Gly Asp Ser Leu Lys Arg Arg Leu
            100                 105                 110

Phe Ala Leu Gly Arg Glu Val Leu Leu Gln Arg Ser Thr Val Glu Lys
        115                 120                 125

Gln Arg Arg Lys Leu Gly Glu Glu Glu Gln Ala Ala Val Leu Leu Met
    130                 135                 140

Ala Leu Ser Tyr Gly Tyr Val Tyr Ala
145                 150

<210>  25
<211>  124
<212>  PRT
<213>  Vitis vinifera

<400>  25

Met Asp Leu Ser Lys Lys Glu Ser Leu Ser Glu Glu Met Asn Glu Ile
1               5                   10                  15

Lys Lys Cys Cys Thr Asp Cys Lys Thr Thr Lys Thr Pro Leu Trp Arg
            20                  25                  30

Gly Gly Pro Ala Gly Pro Lys Ser Leu Cys Asn Ala Cys Gly Ile Arg
        35                  40                  45

Tyr Arg Lys Arg Arg Ser Ser Met Val Gly Val Asn Lys Lys Lys Glu
    50                  55                  60

Arg Met Asn Ser Gly Ser His Asp Leu Ser Glu Thr Leu Lys Gln Ser
65                  70                  75                  80

Leu Met Ala Leu Gly Asn Glu Val Met Met Gln Arg Gln Arg Ser Ser
                85                  90                  95

Val Lys Lys Gln Arg Arg Lys Leu Gly Glu Glu Glu Gln Ala Ala Val
            100                 105                 110

Leu Leu Met Ala Leu Ser Cys Gly Ser Val Phe Ala
        115                 120

<210>  26
<211>  138
<212>  PRT
<213>  Vitis vinifera

<400>  26

Met Asp Pro Gln Glu Gln Val Ser Gly Ser Glu Lys Met Ile Thr Asp
1               5                   10                  15

Ser Gln Pro Asn Lys Ala Glu Gln Leu Lys Arg Ser Cys Ala Asp Cys
            20                  25                  30

His Thr Thr Arg Thr Pro Leu Trp Arg Gly Gly Pro Ala Gly Pro Arg
        35                  40                  45

Ser Leu Cys Asn Ala Cys Gly Ile Arg Tyr Arg Lys Gln Arg Ser Ala
    50                  55                  60

Leu Leu Gly Leu Ala Thr Gly Arg Gly Glu Lys Asn Lys Lys Lys Ile
65                  70                  75                  80

Asn Arg Thr Ser Gly Asn Ser Glu Leu Val Ser Val Lys Leu Arg Leu
                85                  90                  95

Met Ala Leu Gly Arg Asp Met Val Leu Gln Arg Arg Leu Gly Ser Gly
            100                 105                 110

Lys Gln Arg Arg Lys Leu Gly Glu Glu Glu Glu Ala Ala Ile Leu Leu
        115                 120                 125

Met Ala Leu Ser Ser Gly Ser Val Tyr Ala
    130                 135

<210>  27
<211>  309
<212>  PRT
<213>  Vitis vinifera

<400>  27

Met Thr Pro Asn Tyr Leu Asn Ser Pro Pro Pro Pro Pro Phe Pro Leu
1               5                   10                  15

Gln Leu Asn Glu Asp Gln His His Gln Leu Leu Phe Ser Pro Lys Pro
            20                  25                  30

Gln Pro Ser Ser Ser Ser Ser Ser Ser Leu Thr Cys Pro Ile Phe Phe
        35                  40                  45

Ser Pro Thr Lys Glu Gln Gly Gly Cys His Tyr Arg Asp Leu His Gln
    50                  55                  60

Ala Gln Pro Gln Gln Glu Ala His Asp Lys Phe Val Phe Arg Gly Gly
65                  70                  75                  80

Ser Tyr Asp His Pro Thr Leu Glu Ser Glu Ser Asp Asn Gly Leu Lys
                85                  90                  95

Leu Thr Ile Trp Lys Thr Glu Asp Arg Asn Glu Asn His Ser Glu Asn
            100                 105                 110

Gly Ser Val Lys Trp Met Ser Ser Lys Met Arg Val Met Gln Lys Met
        115                 120                 125

Met Ile Ser Asp Gln Thr Gly Ala Gln Lys Pro Ser Asn Thr Ala Leu
    130                 135                 140

Asn Phe Gly Asp His Lys Gln Gln Ser Leu Pro Ser Glu Thr Asp Tyr
145                 150                 155                 160

Asn Ser Ile Asn Ser Ser Asn Ile Asn Ser Asn Asn Thr Ile Arg Val
                165                 170                 175

Cys Ala Asp Cys Asn Thr Thr Lys Thr Pro Leu Trp Arg Ser Gly Pro
            180                 185                 190

Arg Gly Pro Lys Ser Leu Cys Asn Ala Cys Gly Ile Arg Gln Arg Lys
        195                 200                 205

Ala Arg Arg Ala Met Ala Ala Ala Ala Ala Thr Ala Asn Gly Thr Ile
    210                 215                 220

Leu Pro Thr Asn Thr Ala Pro Thr Lys Thr Lys Ala Lys His Lys Asp
225                 230                 235                 240

Lys Lys Ser Ser Asn Gly His Val Ser His Tyr Lys Lys Arg Cys Lys
                245                 250                 255

Leu Ala Ala Ala Pro Ser Cys Glu Thr Lys Lys Leu Cys Phe Glu Asp
            260                 265                 270

Phe Thr Ile Ser Leu Ser Lys Asn Ser Ala Phe His Arg Val Phe Leu
        275                 280                 285

Gln Asp Glu Ile Lys Glu Ala Ala Ile Leu Leu Met Ala Leu Ser Cys
    290                 295                 300

Gly Leu Val His Gly
305

<210>  28
<211>  370
<212>  PRT
<213>  Zea mays

<400>  28

Met Ser Ala Ile Tyr Met Ser Gln Leu Ser Thr Ala Leu Pro Leu Met
1               5                   10                  15

Glu Gly Asp His Gln Asp His His His His His Gln Gly His Phe Gln
            20                  25                  30

Ala Phe Thr Leu Pro Lys Glu Thr Pro Ile Leu Phe Pro Phe Val Ile
        35                  40                  45

Ser Asn Ser Ser Ala Ser Glu Gly Ser Leu Ser Tyr Gly Ser Ala Ala
    50                  55                  60

Asp His His Leu Leu Arg Gln Arg His Gln Ala Met Leu Glu Pro Gln
65                  70                  75                  80

His Met Ile Gly Gly Ser Ser Thr Ala Thr Gly Asn Ser Val Phe Ser
                85                  90                  95

Thr Pro Phe Pro Thr Val Glu Ser Ile Arg Asp Asp Met Ile Glu Pro
            100                 105                 110

Ala Ser Tyr Asp Pro Tyr Asp Met Gly Lys Leu Gln Val Gly Gly Ser
        115                 120                 125

Met Asp Ala Cys Ser Trp Thr Pro Ala Ala Ala Lys Met Arg Ile Thr
    130                 135                 140

Arg Lys Ala Thr Ala Asp Pro Ser Ala Gly Lys Lys Pro Arg Arg Arg
145                 150                 155                 160

Ala Gln Ala Gly Tyr Asp Asp Thr Met Ser Gly Gln Pro Asn Leu Gly
                165                 170                 175

Val Ile Arg Val Cys Ser Asp Cys Asn Thr Thr Lys Thr Pro Leu Trp
            180                 185                 190

Arg Ser Gly Pro Cys Gly Pro Lys Ser Leu Cys Asn Ala Cys Gly Ile
        195                 200                 205

Arg Gln Arg Lys Ala Arg Arg Ala Met Met Ala Ala Ala Ser Gly Ser
    210                 215                 220

Val Ser Ala Val Pro Thr Asp Ser Gly Lys Ala Ser Pro Ser Asn Ala
225                 230                 235                 240

Ala Val Ala Ala Ala Ala Ala His Pro Lys Val Lys Lys Glu Lys Arg
                245                 250                 255

Val Asp Val Asp Arg Ser Leu Pro Phe Lys Lys Arg Cys Lys Val Val
            260                 265                 270

Gln Gln Gly His Gly Ala Ala Val Val Val Ala Ala Pro Ala Ala Ala
        275                 280                 285

Thr Asp Ser Ala Thr Val Val Gln Ala Thr Ala Glu Asp Gly Asp Asp
    290                 295                 300

Asp Thr Cys Pro Ser Arg Asp Leu Leu Val Asp Asp Ile Gly Gly Leu
305                 310                 315                 320

Ile Ser Trp Ser Arg Ser Pro Ala Ala Pro Ala Ser Ala Ala Ala Ala
                325                 330                 335

Thr Cys Ser Phe Arg Ala Ser Pro Ala Leu Pro Val Gln Gln Asp Glu
            340                 345                 350

Ile Thr Asp Ala Ala Met Leu Leu Met Thr Leu Ser Cys Gly Leu Val
        355                 360                 365

Arg Ser
    370

<210>  29
<211>  195
<212>  PRT
<213>  Zea mays

<400>  29

Met Gly Met Glu Ile Asp Met Glu Thr Tyr Pro Asn Leu Asn Thr Ser
1               5                   10                  15

Pro Ser Ala Ala Thr Ala Ser Gly Asp Ala Lys Ala Cys Ala Asn Cys
            20                  25                  30

His Thr Thr Lys Thr Ser Leu Trp Arg Gly Gly Pro Glu Gly Pro Lys
        35                  40                  45

Ser Leu Cys Asn Ala Cys Gly Ile Arg Tyr Arg Lys Arg Arg Gln Ala
    50                  55                  60

Ile Gly Leu Asp Ala Gly Ala Ala Ala Ala Ala Asn Ser Gln Gln Asp
65                  70                  75                  80

Leu Gln Gln Pro Lys Lys Lys Ala Ala Val Asp Pro Gln Gln Gln Asp
                85                  90                  95

Gln His Gln Leu Arg Lys Lys Thr Thr Ala Val Ala Asn Pro Gln Gln
            100                 105                 110

Asp Arg His Gln Pro Arg Lys Arg Ala Ala Ala Ala Ala Ala Ala Thr
        115                 120                 125

Asp Pro Gln His Thr Ser Ile Thr Lys Lys Asp Thr Asp Lys Lys Asp
    130                 135                 140

Gln Gln Val Thr Val Asp Leu His Val Val Gly Phe Gly Lys Glu Ala
145                 150                 155                 160

Thr Phe Lys Gln Arg Arg Arg Met Arg His Asn Lys Cys Met Ser Glu
                165                 170                 175

Glu Glu Arg Ala Ala Val Leu Leu Met Ala Leu Ser Ser Gly Val Ile
            180                 185                 190

Tyr Ala Ser
        195

<210>  30
<211>  1197
<212>  DNA
<213>  Arabidopsis thaliana

<400>  30
atggattcaa attttcatta ctcgatagat cttaacgaag atcaaaacca tcacgaacaa      60

ccctttttct atcctcttgg atcctcttcc tcgcttcatc atcatcatca tcatcatcat     120

catcaagtcc cttctaattc ttcatcttct tcttcgtcca tttcatcgct ctcctcttac     180

ctccctttct tgatcaactc tcaagaagat caacatgttg cctacaacaa cacttatcac     240

gctgatcatc tccatctttc tcaacccctc aaggccaaga tgtttgtggc taacggtgga     300

tcatcagcat gcgatcacat ggtgccaaag aaggagacaa gactgaaact aacgataagg     360

aaaaaagatc acgaagacca accccatcct cttcatcaaa acccgacaaa acccgattca     420

gactccgaca agtggttgat gtccccaaag atgcggttga tcaagaaaac aatcaccaac     480

aataaacagc tcattgatca gactaataat aataatcata aagaaagtga tcactaccct     540

ttgaatcata agactaattt cgacgaggat caccatgaag atcttaattt caagaacgtc     600

ttgaccagga agaccacggc cgcgaccacc gagaatcgct acaatacaat caacgagaac     660

ggttatagta ataacaatgg cgtgattagg gtttgttcgg attgtaacac caccaagact     720

cctctttggc gaagtggacc tcgaggtccc aagtctcttt gtaacgcatg tggtatacgg     780

caaagaaagg caaggcgagc cgctatggcc gcggccgctg cagccggcga ccaagaggtg     840

gcggtagcgc cccgagtgca acaattaccg ctgaaaaaga agttgcaaaa taaaaaaaag     900

agatcaaacg gaggggaaaa atacaatcac tctcctccaa tggtggccaa ggccaaaaag     960

tgcaagatca aagaggaaga ggagaaggaa atggaagcgg aaacggttgc cggagattca    1020

gagatcagca aatctacaac ttcttctaat tcttcgattt cgtcaaacaa attttgcttc    1080

gatgatttga caataatgtt gagcaaaagc tcagcttatc aacaagtgtt cccacaagat    1140

gagaaggagg ctgctgtttt gctcatggct ctgtcgtatg gaatggttca cggttga       1197

<210>  31
<211>  1059
<212>  DNA
<213>  Arabidopsis thaliana

<400>  31
atgggttcca attttcatta cacaatagat ctcaatgaag atcaaaacca tcagcctttt      60

ttcgcttctc ttggatcctc tcttcatcat catctacaac aacaacaaca acaacaacaa     120

cattttcatc accaagcttc ttctaatccc tcttctttga tgtcaccgtc tctttcctac     180

tttcctttct tgataaactc tcgccaagat caagtatatg ttgggtacaa caataacact     240

tttcatgatg ttcttgatac ccatatctcc caacctctcg agaccaagaa ctttgtatct     300

gatggtggtt catcatcaag tgatcaaatg gtgcccaaga aggagacacg actaaaattg     360

acgataaaga agaaagataa tcatcaagac caaaccgatc ttcctcaatc cccaataaaa     420

gacatgacag gaactaactc gctcaagtgg atatcttcga aggtgagatt aatgaagaag     480

aaaaaggcga ttattaccac cagcgacagc agcaaacaac acactaataa cgaccaatcc     540

tcaaacctaa gcaattcgga aagacagaat ggttataaca acgattgcgt gattaggatt     600

tgctccgatt gtaacacaac caagactcct ctttggagaa gtggtccgag aggtcccaag     660

tctctttgta acgcttgtgg aataaggcaa aggaaggcca ggcgggccgc tatggccacg     720

gcaaccgcaa ccgcagtctc tggcgtatcc ccaccggtca tgaagaagaa gatgcaaaac     780

aagaacaaga tatcaaatgg agtttataaa atcttatctc ctttgcccct aaaggtaaac     840

acgtgtaaga gaatgatcac actagaggag accgcattag ccgaggattt ggagacccag     900

agcaactcca cgatgttatc atcttcagac aatatctatt tcgatgatct agcattactg     960

ttgagcaaaa gttcagctta tcagcaagtt ttccctcaag atgagaagga ggctgccatt    1020

ttactaatgg ctctatcgca cggaatggtt cacgggtga                           1059


<210>  32
<211>  489
<212>  DNA
<213>  Hordeum vulgare

<400>  32
atgagttcct cggtcgagaa gggaagcgga tcgctggatc ccggcgagcg cccggcctcc      60

ggcagccagc ccaaggcctg caccgcctgc aacaccacca agacgccgct ctggcgcggc     120

gggccctccg gacccatgtc tctctgcaac gcgtgcggga tccggtacag gaagaagcgg     180

agggaggcgc tgggcctcga cgagccgccc aagaagaggc agcctgctgc ggcagcatcc     240

gctgcagccg cggcggcgtg ctcggaggcc ggcggcgaga gcgcggagcc ggatcagcag     300

cagcagcagc ccaagaagaa gacgacgacg accaagaggg gccgcgaggt ggagctccgc     360

gtggtcgggt tcggcaagga ggtggtgctg aagcagcggc ggcggatgcg gcgcaggcgg 420

cgcctcggcg aggaggagaa ggcggccatc ctcctcatgg cgctctcctc cggcgtcatc 480

tacggctga 489

<210> 33
<211> 546
<212> DNA
<213> Hordeum vulgare

<400> 33
atgtcgccgg cggagatggg gtccgacaag atgaagatga agatgaagat gattgaggtg 60

gaggtccagg cggcggcggc ggatcctgat gactgcacgg cttccggcgc cggcgacccc 120

aagtcctgcg ccgactgcaa caccaccaag acgccgctct ggcgcggagg acccaacgga 180

ccaaagtcgc tgtgcaacgc gtgcgggatc cgctaccgga agaggcggcg ggtggccatg 240

gggctcgacc cggaggccaa gaggaagccc aagagggacg acgccatcaa ttcagccgca 300

gccgcagccg aggcctctac ccagcagcag gaggaggtca ccaagcccac cgacgacgac 360

aaggcggtca gcaccaacaa gactacaaag acgcacaccg ttgagctcca catggtgggg 420

ttcggcaagg acgccgtgct caagcagcgg cgccggatgc gccggaggaa gccgtcgtgc 480

ctgggagagg aggagcgcgc cgccatgctc ctcatggcgc tctcctcggg cgtaatatac 540

gcctga 546

<210> 34
<211> 612
<212> DNA
<213> Nicotiana tabacum

<400> 34
atgcaaagga tgaagaattc agatcataca catatcaaat tgaagttaga agatcagaag 60

aagcaacaat catctcctaa tttggaaact gattacagca gcaacagctc ttccaacaac 120

agtaataaca acaatattcc aattagggtt tgttccgatt gtaacactac taagacccct 180

ctttggagaa gtggtcctaa aggccctaag tccctttgta acgcatgtgg aattcgacaa 240

aggaaagcaa ggagagccat ggcagcagca gctgcagctg cagcaaatgg gacaaatttc 300

acgactgaaa catcaacaac aacaacagca acagcaatga agataaaagt gcaacagaaa 360

caaaagataa ctacaaaagt gaataacaat catgttgtac ccttcaagaa aaggtgcaaa 420

ttcataacat ctactactgc agctgttgga tcatcttcat ctactattaa taatgcacaa 480

aagaagattt gtttcgagga tttcttcgtc aacttgagca acaatttggc gttacatcgc 540

gtttttccac aagatgagaa ggaagctgca attttgctaa tggcactatc tagtggcctt 600

gttcatggtt ga 612

<210> 35
<211> 1062
<212> DNA
<213> Oryza sativa

<400> 35
atgtctacta tctacatgag ccagctacct gctactctcc ctctaatgga gggggatcag 60

gatcaggggc tctacccagc cttccataga gcaaaggacc ctcctatctt gttccctttc 120

atgatcgaca gcgccgtcga gcaccaaggg caaatctatg gagatcaggg cttgaggagg 180

cagcaggttt tgggtgaatc caatcaacag ttcaatgatc acatgatgat gggcggatca 240

gatgtcttcc tcacaccgtc tccgttccga ccaaccatcc aaagcatcgg cagcgacatg 300

atccagcgat catcttatga tccatacgat atcgagagta acaacaagca gcatgccaat 360

ggatcaacca gcaagtggat gtcgacgccg ccaatgaaga tgaggatcat aaggaagggg 420

gcggcaaccg atcctgaggg cggggcggtg agaaagccaa ggagaagagc acaagcgcac 480

caggatgaga gccagcaaca actgcagcaa gctttgggtg tcgttagagt gtgctcggac 540

tgcaacacca ccaagacccc cttgtggaga agtggtcctt gtggccccaa gtccctttgc 600

aacgcgtgtg gcatcaggca aaggaaggcg cggcgggcga tggccgctgc tgccaacggc 660

ggagcggcgg tggcgccggc aaagagcgtg gccgcggcgc cggtgaacaa taagccggcg 720

gcgaagaagg agaagagggc ggcggacgtc gaccggtcgc tgccgttcaa gaaacggtgc 780

aagatggtcg atcacgttgc tgctgccgtc gctgccacca agcccacggc tgctggagaa 840

gtagtggccg ccgctccgaa ggaccaagat cacgtcatcg tcgtcggtgg cgagaacgcc 900

gccgccacct ccatgccggc acagaacccg atatccaagg cggcggcgac cgccgctgcc 960

gccgccgcct ctccggcgtt cttccacggc ctccctcgcg acgagatcac cgacgccgcc 1020

atgctgctca tgaccctatc ctgtggcctc gtccacagct ag 1062


<210> 36
<211> 411
<212> DNA
<213> Oryza sativa

<400> 36
atggattcct cgtcggtcga gaaggggagt gggtcgatag atccggacga gcgcacggcc 60

tccggcgagc ccaaggcgtg caccgactgc cacaccacca agactccgct ctggcgcggc 120

ggcccctccg gccccaagtc gctgtgcaac gcgtgcggga tccggtaccg gaagaagaga 180

cgggaggcgc tggggctcga cgccggcgag ggcggcgcgg agcggcagga gaagaagaag 240

agcaagaggg agagagggga ggaggtgacc atggagctcc gcatggtggg gttcgggaag 300

gaggtggtcc tgaagcagcg gcggcggatg cggcggagga gacgcctcgg cgaggaggag 360

aaggcggcca tcctcctcat ggccctctcc tccggagtca tctacgcctg a 411

<210> 37
<211> 456
<212> DNA
<213> Oryza sativa

<400> 37
atgggttctt ccgatcagaa ggtgatcggg atcgccgcgg cggcagcggc ggcggcggag 60

gaggctggga ggcggtgctg cgtcgaatgc ggcgccacca ccacgcccat gtggcgcggc 120

ggcccgaccg gccccaggtc gctttgcaat gcgtgcggga tccggtacag gaagaagagg 180

aggcaagagc taggcctaga caagaagcag caacaagaac atcatcctca tcatcatcaa 240

caacagcagc agcagtatca gcggcagcaa cagcaacagc aacaagaaga tcacagtgat 300

gcagcaagtt cagtaaaaga cagtagcagc agcagtagca acaaaagcag cagcctccaa 360

gtggtgaaga aaaggagggt cctgatggga gtggaggagg ctgcaatctt gctcatggcc 420

ttgtcctcat cttcacccac actactacat ggctag 456

<210> 38
<211> 1044
<212> DNA
<213> Oryza sativa

<400> 38
atgtctacca tctacatgag tcagctctca gctgctctcc ctctcatgga gggggagcac 60

caccatcacc accaggatca tcaccaaggc cacttccaag ccttctccct gcagcctaag 120

gatcccccag tcttattccc ctttgtgatc agtagaagaa gcagcagcag cagccctagc 180

gacagcacca ctctaagcta tggttcagac catcacttga cacagcagca gcagcatcag 240

catcaagcca tgcttgagcc ccaaaatatg attggaggat catccgctgg catctttgcg 300

acgccgttcc cgaccgtcaa gagcatccgc gacgacatga tcgagcggtc gcagttcgat 360

ccatacgata ccgagaagct gcaggcgagc tgcgggttag ccaaggtcgt cgccggcggc 420

aagtggagcg cggtgccagc ggccaagatg aagatcacga ggaagatggg tgagccgtcg 480

tccggtgtca ctggcggggc tgcgacgacg gtggcgccga agaagccgag gaggaggccg 540

gcgcaggcgt acgaggatca cggccatggc ggcgccatgg gccaagcttt tggcgtgatt 600

agggtgtgct ccgactgcaa caccaccaag actcccttgt ggaggagtgg cccgtgcggc 660

cccaagtcgc tttgcaacgc gtgcggcatc aggcagagga aggcgcggcg ggcgatgatg 720

gcctccggac taccagcgtc ccccaacgcc gccggcccca aggcggccgc acatagcggc 780

gccacaaacg cagccgccgc agctgccatg gaggagacgg ccgagtccgc caccgtcgcc 840

ccgcccccgg cgccgacgac gaggggtggt actctcgtcg acagcatcgg gctcagctgg 900

agcaagaccc atgccgccgc caccgcctcc tgcagcttcc ggccgtcacc ggtggctccc 960

ggcttcgcgg cggcggtgca ggacgagatc actgacgccg ccatgctgct catgacgctg 1020

tcctgcgggc ttgtccggag ctga 1044

<210> 39
<211> 444
<212> DNA
<213> Populus trichocarpa x Populus deltoides

<400> 39
atgatcaagg atttgcgcac aaaaggacca gagtcagagg acatggataa tactcatcca 60

agcaagtgta atgagatcaa aagaagatgc acagattgcc aaacaacaag aaccccatgt 120

tggcgaggtg gcccagctgg tcccaggaca ctgtgcaatg cgtgtgggat cagacagagg 180

aagaagagga gggctcttct tggcttcgac aaaggaggac cggagcgcag cagagagaag 240

atggctaagg gtagcaatag tagcaagcta ggagtttcat tgaatcttgg tttgatgggg 300

tttaaaagag atgggatgtt tcaggaagat tggaagataa aactgggaga ggaagaacag 360

gctgccatac tcttgatggc tttatcttgt gggtcggtct tgccttggta caagaaagat 420

gaaggaaagc atttttatcc atag 444

<210> 40
<211> 372
<212> DNA
<213> Populus trichocarpa

<400> 40
tcagaggaca tggataatac tcatccaagc aagtgtaatg agatcaaaag aagatgcaca 60

gattgccaaa caacaagaac cccatgttgg cgaggtggcc cagctggtcc caggacactg 120

tgcaatgcgt gtgggatcag acagaggaag aggaggaggg ctcttcttgg cttggacaaa 180

ggaggaccgg agcgcagcag agagaagatg gctaagggta gcaatagtag caagctagga 240

gtttcattga atctagattt gatggggttt aaaagagatg ggatgtttca ggaagattgg 300

aagagaaaac tgggagagga agaacaggct gccatactct tgatggcttt atcttgtggg 360

tctgtctgtg ct 372

<210> 41
<211> 705
<212> DNA
<213> Populus trichocarpa

<400> 41
atgtacaaca tatcacatgg cggatcaagc tcttcttttc aacctgaggt gaatgatcac 60

aattataata gcaatttcca taacttatcc tcatctaaaa tggaggatgg agctgaagaa 120

agtggtgaaa gttcagtcaa atggatgccc tcgaagatga ggctgatgca aaaaatgacc 180

aactcgaatt gctcagaaac tgatcatatg ccaatgaaat ttatgctcaa gtttcacaat 240

cagcaatatc agaataacga gatcaactcc tccagcaaca gcaacagcaa tattagagtc 300

tgctcggatt gtaacactac ctcaacccca ctttggagga gcggtccgcg cggccctaag 360

tcactttgca atgcctgcgg gattcggcaa aggaaggcca gacgggctat ggcagcagcc 420

gcagcggctg caaatggcac agttattgcc atcgaggcat cgtcatctac gaggagcact 480

aaggtaaaca acaaggtaaa gaaatcgagg acgaaccatg tttcgcaaaa caagaaactg 540

agcaagcctc ctgaatcatc tcttcagagt caaaagaagc tttgtttcaa aaatctcgca 600

ttaagtttga gcaaaaatcc agccttacaa caagtgcttc cgcatgatgt ggaagaagct 660

gcaatccttc taatggaact atcttgtggc tttattcaca gctga 705

<210> 42
<211> 366
<212> DNA
<213> Populus trichocarpa

<400> 42
atggatagta ctcaatcaag caagggtaac gagatcaaaa gaagatgcat ggattgccaa 60

actacaagaa ccccatgttg gcgaggcggt ccggctggtc ccaggacact gtgcaatgca 120

tgtgggatca gacagaggaa gaagaggaga gcacttcatg ggtcggacaa aggaggagcg 180

gagaggagta aaaataagat tgctaaaagt agcaatagta gtaagctagg agtttcattg 240

aagctagatt tgatgggttt tagaagagat gggatattgc aggaagattg gaagagaaaa 300

ctgggagagg aagaacaggc tgctatactc ttgatggcct tatcttgtgg cttggtccgt 360

gcttag 366

<210> 43
<211> 378
<212> DNA
<213> Populus trichocarpa

<400> 43
atgagtagca attcacaaga aacagagagc ccactaaaaa agacttgtgc tgattgtggt 60

acttcaaaga ctccactttg gagaggtggt ccagctggtc caaagtcgct gtgtaatgct 120

tgtgggatta gaagcagaaa gaagaagagg gatattttag ggttgaataa aggggcagca 180

aatgacaaga gagcaaaaaa gggaagcaat aataatggct caagcaataa caacaacaac 240

aagcagttag gtgatggatc aaagcagaga ttattggcac tgggcagaga agttttgatg 300

caaagaagaa agctgggtga agaagagcaa gctgcagtgt tgttgatggc gttgtcttat 360

gggtctgttt atgcttaa 378

<210> 44
<211> 378
<212> DNA
<213> Populus trichocarpa

<400>  44
atgagtagca aatcactaga aaccgagagc ccacagaaga agacttgtgc tgattgtggc      60

acttcaaaga ctccactttg gagaggtggt ccagctggcc ctaagtcact gtgtaatgct     120

tgtgggatta ggagcaggaa gaagaagagg gatatattag ggttgaataa gggaggggca     180

gcagcaaatg acaagagagc caagaaaggg agcactaaca atggctcaag tgatgggtta     240

aagcagagat tactggctct gggtagagaa gtcttggtgc aaggatcaac agtggagagg     300

cgaaggagga agctaggtga agaagagcaa gctgcagtgt tattgatggc cttatcttat     360

ggctctgttt atgcttga                                                   378


<210>  45
<211>  939
<212>  DNA
<213>  Ricinus communis

<400>  45
atgactccta cttatcattc atcttttcct ccttttacta tagaccttaa tgaagatcaa      60

catcaccacc agctcatctt ctgctcgaaa actactactg aagatgcatc ttcatcttct     120

tctatatcct accccatttt tattaaccca cctcaagaag aagttggata ttaccataaa     180

gagttgcagc ctttacacca tcaggaagtg gataacatat atgcttcgca tggtagatca     240

tgggatcatc gaataattaa gaacgagaac gagaatggcc aagaattatc tgtttgcaag     300

aaagaagaca aaagtactag tattgaagat caaagagaca acagttccgt taagtggatg     360

tcttcaaaga tgaggttgat gaggaagatg atgactacgg atcaaacggt caatactaca     420

cagcatacat cttctatgca caagcttgag gataaagaga agagccgatc attaccttta     480

caagatgatt atagcagcaa aaacttgtct gacaacagta ataacacaat tagggtttgt     540

tctgattgta acactacaaa gacccctctt tggagaagtg gaccacgagg tcctaagtca     600

ctttgcaacg cctgtggaat tcgacaaaga aaggcgaggc gagccttggc tgcagctcaa     660

gcaagcgcaa atgggacaat ttttgctcct gacacagcag ccatgaagac taacaaggtg     720

caaaacaaag agaagaggac aaacaatagt catcttccat tcaagaaaag gtgcaaattt     780

acagcccaat ctcgaggcag cagaaagaaa ctttgttttg aagatttatc atcaacgatc     840

ttgagcaaga attcagcttt ccaacaactt tttcctcaag atgagaagga agctgcaatt     900

ctactaatgg ctttatctta tggcctggtt catggttga                            939


<210>  46
<211>  450
<212>  DNA
<213>  Ricinus communis

<400>  46
atgttggatc caagtgaaaa aatattggag tttgaggata tgagtagcaa atcagcagaa      60

ggagaaaatc aacaaaagaa aagttgtgct gattgtggta ctactaagac accgctttgg 120

aggggtggcc cagctggccc taagtcactg tgtaacgctt gtggtattag gagcaggaaa 180

aagaaaagag acagtctggg tctgaataga gcatcatcaa atcctgacaa gaaatcaaga 240

aaacacagtt caagcaatgg aagcagcaac aatcataata gcaataacag taataggtta 300

ggagatgggt taaagcaaag actgttggct ttgggtagag aagtattgat gcagagatcg 360

agtgtggaga aacagagaag aaagttagga gaagaagaac aagctgctgt gctattaatg 420

gctttatctt atggctctgt ttatgcttag 450

<210> 47
<211> 561
<212> DNA
<213> Ricinus communis

<400> 47
atgccctcaa agatgagaat aatgcaaaaa atgatgaact ctaattgctt tgagttcaat 60

gataagccag tgaaatttac agtcaagttt caagatcacc aacaatatca agctactaac 120

aatgagataa actcatcctg cagcaacggc aacaacaata ttagagtctg ctccgactgc 180

aacaccacca ctacccctct ttggaggagt ggccctcgtg gccctaagtc cctttgcaat 240

gcttgtggga tccgacaaag gaaggccaga cgtgcaatgg cggctgcagc agcaattgca 300

atggagacat catctacaaa ggcggctaag gttaaggaaa aaaagtcgcg cacaggccat 360

gcatcacaat gcaagaaact gtgcaagcct cctgatcatc caccacctcc ttataatcag 420

ggccaaaagc caaaagttag cttcaagaat ctggcgctaa gtttaagcaa caattctgct 480

cttcaacgag tgtttccaga ggatgttgaa gaagcagcaa cgctgcttat ggaattatca 540

tgtggtttta ttcacggttg a 561

<210> 48
<211> 474
<212> DNA
<213> Sorghum bicolor

<400> 48
atggactcct ccgctgagaa gcaggggagc gggacgctgg atccggacga gcgcccgccg 60

gcgtccggcg agactaaggc ctgcaccgag tgccacacca ccaagacccc gctctggcgc 120

gggggaccct gcggacccat gtcgctgtgc aacgcgtgcg ggatccggta ccggaagaag 180

agacgggagg cgatgggcct ggactccagc agcaaggccg gcggcggcac cgagcagcag 240

cagcagcgga agaagaaggc caccgcggcc gcggccgccg cggcggcgtc caagcgggag 300

agggagaggt cgaaggaggc cgacgaggtc accgtggagc tccgcgccgt cgggttcggc 360

aaggaggtgg tgctgaagca gcggcggcgg atgcgccgga ggcggcgcct cggcgaggag 420

gagcgcgcgg ccttcctcct catggcgctc tcctccggcg tcgtgtacgc ctga 474

<210>  49
<211>  654
<212>  DNA
<213>  Sorghum bicolor

<400>  49
atggaggtgg agatggaggt ggagatgggg acggatccca atcccaatcc caatcccaat      60

cctaacactt ccccctcggc cgccgacgac gccgccaccg cctcaggcga ggccaaggcc     120

tgcgccgact gccacaccac caagacgccg ctctggcgcg gaggacccga gggacccaag     180

tcgctctgca acgcctgcgg catccgctac cgcaagagga ggaggcaggc gctcggtctc     240

gacgccgcgg ccgccgactc acagcaagat cagcagcagc cgaagaagaa ggccaccgcc     300

gatccgcagc agcaggatca gcatcacctg agaaagaagg ccgccgatcc acagcagcag     360

gatcagcatc acctgagaaa gaaggcagct gccgctgcca ccaccgatcc acagcagcag     420

gatagaaaga aggccgctgc cgccagcagc accaacaaga aagacaaaga taaggaggac     480

aacaagaaga agaaggatca gcaagtcacc gtggagctcc gcgtggtggg gttcggcaag     540

gaggtgatgc tcaagcagcg ccgccggatg cgccgcaaga agtgcatgag cgaggaggag     600

agggccgccg tcctccttat ggcgctctca tcgggcgtca tctacgcctc atga           654

<210>  50
<211>  1116
<212>  DNA
<213>  Sorghum bicolor

<400>  50
atggagggag agcaggatca ggatcaggga ctctttcggc cggcgccctt ccatgtcgca      60

aaggaccctc ccatcttgtt cccgttcatg atcaccaatc ctgtggatca gctccaaggg     120

caaagcagca gctatggaga tcagcacttg aggcagcatg ttttgtctga atctactcat     180

caacagttca ccgatcggat gatgatgagc ggatcagaag tcttccctac tatgccatcg     240

ccgttccggc agactatcca aagcatcgat ggtgacatga tccagcgatc tgcgtatgat     300

ccatatgata cagagaataa taagcacacc gaaggattgc gcagcggcgg cggcggcggc     360

ggctggacag tgtcgtcgtc accgccagcg aagatgaaga tcatgcggag gaaggcaacg     420

agcgagtatc ccgagggcgg tggtggggga gcaagaaagc caaggagaag accacaagcg     480

caccaccaag atgctgagag ccagctactc cagcagcagc agcaggctat gggtggtgtc     540

gttagagtgt gctccgactg caacaccaca aagaccccct tgtggaggag tggtccttgt     600

ggccccaagt ctctttgcaa cgcatgtggc ataaggcaaa ggaaggcgag gcgggcgatg     660

gcggcagccg cggcagctgc tgctgccagc agcaacggag gcgctccaca agcggccacc     720

gagcagcagc cgaagccggc caagaaggag aagaggtcgg acatcgtcga ccggtcgtcg     780

ctgccgttca agaagcggtg caagcagacg atggtagttg tcgatcacgc tgctggtgtt 840

gcagccactg aagcgacgcc gccgccgccg ccgccagaaa ccgctgctct gaccaaggat 900

caggcggatg tggatcacgt ctccagtgac aagaagcagg ttaatggcca tggcgcgacg 960

agtctggaga gcagcaagat cgtcgccact cctcctcccc aggcgatgtc gtcgtttcat 1020

gccttcccgg ctgacgagat cactgacgcc gccatgctgc tcatgaccct gtcctgtggg 1080

cacgctcgtc tgcagctagc catggcttct agctag 1116

<210> 51
<211> 1161
<212> DNA
<213> Sorghum bicolor

<400> 51
atgtctgcca tctacatgag ccagctctcc actgctctcc ctctcatgga gggagatcac 60

caccaccacc accaccacca ccaccaaggc cacttccaag ccttcacgct gccaaaggat 120

cccccaatcc ttttcccctt tgtgatcagc aatagtagcg ccagcgaaag cagtctgagc 180

tatggatcgg cagatcatca cttgttgagg cagcatcgtc aaactatgct cgagccccaa 240

catatgattg gtggatcgtc atcagctgcg agtagtgtct ttgcgacgcc gttcccgact 300

gtggagagca tccgtgacga catgatcgag cctgcctcgt acgatccata cgatatgggg 360

aagctgcacc aggtggtcgg tggcgggtcg tcgatggatg cttgcagctg gacgccgccg 420

gcggccaaga tgaggatcac gaggaaggcc actgccgccg atcccagtgg tgccgggaag 480

aagccgagga gaagggcgca tcaggcagca gggtacgacg ccgacatcaa catgagcggc 540

caaccaaatt tgggtgttat tagggtgtgc tccgactgca acaccaccaa gacgcccttg 600

tggaggagtg gtccttgcgg ccccaagtcg ctctgcaacg cgtgcggtat caggcagcgg 660

aaggcacggc gggcgatgat ggccgccgcg tctacctccg ggccagcagc agtgcccgcc 720

accgacagcg ataaggcctc gccgagcaac gccgccgggg ccgcagcagc acaccccaag 780

gtgaagaagg agaagagatc agtggacgtg gaccggtcgc tgccgttcaa gaaacggtgc 840

aaggtggtcc aggtccagca ggatcacgct gcggccgtcg cagctcctgc cgcggccact 900

gacaggccgg ctgtcgtcgt gcagcaggcg gccaccgccg ccgaggttgg tgacgacgac 960

gcctgtccga gcagggacct gcttgtcgac gacattggtg ggctcatcag ctggagcagg 1020

agtccgccgg cggctcccgc atctgcagat gctgcctcct gcagtttccg ggcgtcgccg 1080

gcgttgccgg tgcagcagga cgagatcacg gacgctgcca tgctgctcat gacgctgtcc 1140

tgcgggcttg tccgaagctg a 1161

<210> 52
<211> 921
<212> DNA

<213>  Vitis vinifera

<400>  52
atgactccag tttttctgaa cacttcatca tcttctcctt ttcctgctct ggagcttaaa      60

gaagatcatc aacacttcca gctccttttt agtacaaacc caccatcata ccaagcttct     120

tcctctcacc catgtcctag tttcttcaac tcaagtactc aatctcagag aggagatcat     180

agccctagag acccccaaca gcatgaagat aaggatgata agtacatttc ccatggagga     240

tgtggtgaaa gccaagtatt ctcatcttct tcactcctgc agcccatggc ggatgacaat     300

aagagcagcc acaaattgtc ggtttttaag aaggaagaag gagatgaagg taataaaagt     360

actgagaaat ggatgtcttc aaagatgagg ctgatgagaa aaatgatgaa ctcggattgc     420

actacagcga aaatcgagca gaaggttgaa gatcatcagc agtgggacaa tattaatgag     480

ttcaactctt ccaacaatac tagtaatatc ccaattagag tctgcagtga ttgtaacaca     540

accaaaaccc ctctttggag gagcggtcct agaggtccca agtcactttg caatgcctgt     600

ggaattaggc aaaggaaggc gagacgagcc atggcagcag cggcagcagc agcagcgaat     660

ggcacagccg ttgggaccga gatatcgcct atgaagatga agctgcccaa caaggaaaag     720

aagatgcata caagcaatgt agggcaacag aagaagctct gcaagccccc ttgtcctcct     780

cccaccgaga agaagctttg cttcgaagat ttcacttcga gtatttgcaa gaactcaggt     840

tttagacgag tgttccctcg ggatgaagaa gaagccgcga tcctcctaat ggccttatct     900

tgtgaccttg tttacagttg a                                               921


<210>  53
<211>  462
<212>  DNA
<213>  Vitis vinifera

<400>  53
atggtggatc tgagtgagaa agggtcagag tcggaagaca tgaacaacaa aaacccagat      60

gctgtttcgt cggctgagag ccaggttaac gagccgaaga agacctgcgc tgattgtggc     120

accaccaaaa cccctctctg gagaggcggt ccagctgggc ctaagtctct gtgcaatgca     180

tgtggtatca gaagcaggaa gaagagaaga gccttcctgg ggttgaacaa aggatcaaca     240

gatgatagaa aagcaaagag aagcagcaac cactcccata acaatggtgg cggcaacggg     300

aacaataaat tgggggactc gctgaagagg aggctcttcg cattgggaag agaggtgttg     360

ttgcagagat caacagtgga gaaacagagg aggaagctgg gagaagaaga gcaagcggcc     420

gtactgttaa tggctctgtc ttacggctac gtctatgctt aa                        462


<210>  54
<211>  375
<212>  DNA
<213>  Vitis vinifera

<400> 54
atggatctga gcaaaaagga atcattgtct gaggaaatga atgagatcaa gaaatgttgt 60

actgattgca agaccaccaa gacgcccctg tggagaggtg ggccagctgg gcctaagtca 120

ctctgcaatg catgtgggat cagatacagg aagaggagga gttccatggt gggtgtgaac 180

aaaaagaaag agagaatgaa cagtggtagc catgatttga gtgaaacttt gaagcagtca 240

ctcatggctt tggggaatga ggtgatgatg cagaggcaga gatcttcagt gaagaaacag 300

aggaggaagt tgggggaaga agaacaagca gctgtactgt tgatggcact ctcatgtggc 360

tctgtttttg cctag 375

<210> 55
<211> 417
<212> DNA
<213> Vitis vinifera

<400> 55
atggatcccc aagaacaagt ttcggggtct gagaaaatga ttacagattc tcaaccaaac 60

aaggccgagc agctcaagag aagctgtgcg gattgccaca ccacaagaac acctctatgg 120

agaggcggac cagctgggcc aaggtcgcta tgtaatgctt gtgggatcag atacagaaaa 180

cagaggagcg cccttctggg gttggccaca ggaagaggag aaaagaataa gaaaaagatt 240

aacagaacca gtggcaatag tgagcttgtt tcagtgaagc tcagattgat ggctttggga 300

agagatatgg tgttgcagag aagattggga tctggaaaac agaggaggaa gttgggagaa 360

gaagaagaag ctgcaatact gttgatggct ctctcatctg gttctgtata cgcttag 417

<210> 56
<211> 930
<212> DNA
<213> Vitis vinifera

<400> 56
atgactccaa attacctaaa ttcaccacct cctcctcctt ttcctttaca gctcaatgaa 60

gatcaacacc atcagcttct cttcagtcca aagcctcaac cttcttcttc ttcatcttct 120

tctctaacat gtcctatttt cttcagccca actaaggagc aaggtggatg tcactatagg 180

gatttacacc aggcacaacc tcaacaagag gctcatgata aatttgtttt ccggggtgga 240

tcatatgatc atccaacact ggagagtgag agtgacaatg gcctcaagtt aaccatttgg 300

aagacagaag acagaaatga aaatcacagt gaaaatggct cagttaagtg gatgtcttca 360

aagatgaggg tgatgcagaa gatgatgatc tcggatcaaa ctggtgccca aaaaccaagc 420

aacactgcac tcaattttgg agatcacaag cagcaatcct tgccttctga aaccgattac 480

aatagcatca attcctctaa tatcaacagc aacaacacaa ttagggtttg tgcagattgt 540

aacacaacta agacccctct gtggaggagt ggcccaagag gccctaagtc tctctgcaac 600

gcctgcggaa tcaggcaaag gaaggctaga cgggccatgg ctgctgctgc tgcaactgct 660

aatggcacaa ttcttccaac caacacagca cccacaaaga ccaaggccaa gcacaaagac 720

aagaagtcga gcaatggtca tgtttcacac tacaagaaac ggtgcaaact ggctgcggcc 780

ccatcttgtg aaacaaagaa gctttgtttc gaggacttca ccataagctt gagtaagaat 840

tccgctttcc accgagtttt cctccaagac gagatcaagg aagcggcgat cctgctaatg 900

gctctatctt gcggcctcgt ccatggttga 930

<210> 57
<211> 1113
<212> DNA
<213> Zea mays

<400> 57
atgtctgcca tctacatgag ccagctctcc actgccctcc ctctcatgga gggagatcac 60

caagatcatc atcaccacca ccaaggccac ttccaggcct tcaccctgcc aaaggaaaca 120

ccgatccttt tcccctttgt gatcagcaat agtagcgcca gcgaaggcag tttgagctat 180

ggatcggctg cagaccatca cttgttgagg cagcgtcatc aagctatgct ggagccccaa 240

catatgattg gaggatcatc aactgcgact gggaatagtg tcttctcgac gccgttcccg 300

accgtggaga gcatccgcga cgacatgatc gagcctgcct cgtacgatcc atacgatatg 360

gggaagctgc aggtcggcgg gtcgatggat gcttgcagct ggacgccggc ggcagccaag 420

atgaggatca cgaggaaggc cactgccgat cccagtgcgg ggaagaagcc aaggagaagg 480

gcgcaagcag ggtacgacga caccatgagc ggccaaccga atttgggtgt tattagggtg 540

tgctccgact gcaacaccac caagacaccc ttgtggagga gtggtccttg cggccccaag 600

tcgctctgca acgcgtgcgg tatcaggcag cggaaggcga ggcgggcgat gatggccgcc 660

gcctccggct cggtgtcagc agttcccact gacagcggca aggcctcgcc cagcaacgcc 720

gccgtggccg cagcagcagc acaccccaag gtgaagaagg agaagagagt ggacgtggac 780

cggtcgctgc cgttcaagaa acggtgcaag gtggtccagc aaggtcacgg tgcggccgtc 840

gtcgttgcag ctcctgctgc tgccaccgac agcgcgactg tcgtgcaggc caccgccgag 900

gatggtgatg acgacacctg tccgagcagg gatctgcttg tcgacgacat tggtgggctc 960

atcagctgga gcaggagtcc ggcggcaccc gcctcagcgg ctgcggccac ctgcagcttc 1020

cgggcgtcgc cggcgttgcc cgtgcagcag gacgagatca cggacgctgc catgctgctg 1080

atgacgctgt cctgcgggct tgtccggagc tga 1113

<210> 58
<211> 588
<212> DNA
<213> Zea mays

<400> 58
atggggatgg agatagacat ggagacgtat cccaatctta atacttcccc ctcggccgcc 60

actgcctcag gcgacgccaa ggcctgcgcc aactgccaca ccaccaagac gtcgctctgg 120

cgcggaggac cggagggccc caagtccctc tgcaacgcct gcggcatccg ctaccgcaag 180

aggaggcagg cgatcggtct cgacgcagga gcggccgccg ccgccaactc acagcaagat 240

ctgcagcagc caaagaagaa ggccgccgtc gatccgcagc agcaggacca gcatcagctg 300

agaaagaaga caacagcggt cgctaatcca cagcaggatc ggcatcagcc gagaaagaga 360

gcagctgccg ctgccgctgc caccgatcca cagcacacca gcatcaccaa gaaagacacg 420

gacaagaagg atcagcaagt caccgtggat ctccacgtgg tggggttcgg caaggaggcg 480

acgttcaagc agcgccgccg gatgcgccac aataagtgca tgagcgagga ggagagggcc 540

gctgttcttc ttatggcgct ctcatcgggc gtcatatatg catcatga 588